# EUROPEAN PATENT APPLICATION

(11) **EP 2 952 582 A1**
(43) Date of publication of application: **09.12.2015**
(21) Application number: 14746442.4
(22) Date of filing: 29.01.2014
(51) Int. Cl.: C12N 15/09, C12P 19/34, C40B 50/06

(54) **FLEXIBLE DISPLAY METHOD**

(30) Priority: 30.01.2013 JP 2013015423
(71) Applicant: PeptiDream Inc., Tokyo 153-8904 (JP)
(72) Inventor: REID, C. Patrick, Tokyo 153-8904 (JP); SASAKI, Tooru, Tokyo 153-8904 (JP); MURAKAMI, Hiroshi, Tokyo 155-0031 (JP)
(74) Representative: Keirstead, Tanis Evelyne
(86) International application number: PCT/JP2014/051907
(87) International publication number: WO 2014/119600

(57) **Abstract**

An improved method used in selecting a useful protein, peptide, peptide analog by an evolution molecule engineering is provided. A transcription-linker association-translation coupling reaction system characterized by incorporation of a template DNA library to enable a step of forming translation product/linker/mRNA complexes through transcription of a template DNA library to mRNAs, association of mRNAs with linkers, translation of mRNAs, and binding with translation products to be automatically performed in a reaction system, comprising factors necessary for transcription, factors necessary for translation, and linkers.

### SEQUENCE LISTING

<110> PeptiDream Inc.
<120> Flexible Display
<130> FA1562-13098
<150> JP2013-015423
   <151> 2013-01-30
<160> 36
<170> PatentIn version 3.1
<210> 1
   <211> 44
   <212> DNA
   <213> Artificial
<220>
   <223> T7SD8M2.F44
<400> 1
   atactaatac gactcactat aggattaagg aggtgatatt tatg 44
<210> 2
   <211> 41
   <212> DNA
   <213> Artificial
<220>
   <223> G5S-4an21.R41
<400> 2
   cccgcctccc gccccccgtc ctagctacct cctcctccac c 41
<210> 3
   <211> 36
   <212> DNA
   <213> Artificial
<220>
   <223> G5S-4an13.R36
<400> 3
   tttccgcccc ccgtcctagc tacctcctcc tccacc 36
<210> 4
   <211> 20
   <212> DNA
   <213> Artificial
<220>
   <223> G5S-4.R20
<400> 4
   tagctacctc ctcctccacc 20
<210> 5
   <211> 21
   <212> DNA
   <213> Artificial
<220>
   <223> Biotin-DNA
<400> 5
   cccgcctccc gccccccgtc c 21
<210> 6
   <211> 21
   <212> DNA
   <213> Artificial
<220>
   <223> Competitor DNA
<400> 6
   cccgcctccc gccccccgtc c 21
<210> 7
   <211> 16
   <212> DNA
   <213> Artificial
<220>
   <223> Puromycin-linker.an13
<400> 7
   ctcccgcccc ccgtcc 16
<210> 8
   <211> 21
   <212> DNA
   <213> Artificial
<220>
   <223> Puromycin-linker.an21
<400> 8
   cccgcctccc gccccccgtc c 21
<210> 9
   <211> 23
   <212> DNA
   <213> Artificial
<220>
   <223> SD8-NNK
<400> 9
   tagctacctc ctcctccacc gca 23
<210> 10
   <211> 22
   <212> DNA
   <213> Artificial
<220>
   <223> NNK-CG5S4
<400> 10
   cataaatatc acctccttaa tc 22
<210> 11
   <211> 72
   <212> DNA
   <213> Artificial
<220>
   <223> SD8T7G5S4.R72
<400> 11
   tagctacctc ctcctccacc acccatttgc tgtccaccag tcatgctagc cataaatatc 60
   acctccttaa tc 72
<210> 12
   <211> 69
   <212> DNA
   <213> Artificial
<220>
   <223> SD8No38revG5S4.R69
<400> 12
   tagctacctc ctcctccacc gcaagtataa ttctcacgca gaaaccacat aaatatcacc 60
   tccttaatc 69
<210> 13
   <211> 69
   <212> DNA
   <213> Artificial
<220>
   <223> SD8No38ranG5S4.R69
<400> 13
   tagctacctc ctcctccacc gcaataccaa ttcagagtct caaaacgcat aaatatcacc 60
   tccttaatc 69
<210> 14
   <211> 75
   <212> DNA
   <213> Artificial
<220>
   <223> SD8No41revG5S4.R75
<400> 14
   tagctacctc ctcctccacc gcaagactgc caatcaggcc aagcaataaa ccacataaat 60
   atcacctcct taatc 75
<210> 15
   <211> 75
   <212> DNA
   <213> Artificial
<220>
   <223> SD8No41ranG5S4.R75
<400> 15
   tagctacctc ctcctccacc gcaaaaagca atccaccacc aaggagactg atccataaat 60
   atcacctcct taatc 75
<210> 16
   <211> 24
   <212> RNA
   <213> Artificial
<220>
   <223> C+GGGGGS spacer
<400> 16
   ugcgguggag gaggagguag cuag 24
<210> 17
   <211> 7
   <212> PRT
   <213> Artificial
<220>
   <223> C + spacer pepide
<400> 17
   Cys Gly Gly Gly Gly Gly Ser
   1 5
<210> 18
   <211> 21
   <212> RNA
   <213> Artificial
<220>
   <223> An21 mRNA annealing sequence
<400> 18
   ggacgggggg cgggaggcgg g 21
<210> 19
   <211> 44
   <212> RNA
   <213> Artificial
<220>
   <223> spacer and annealing sequence
<400> 19
   ugcgguggag gaggagguag cuaggacggg gggcgggagg cggg 44
<210> 20
   <211> 18
   <212> RNA
   <213> Artificial
<220>
   <223> stop and annealing sequence to An13
<400> 20
   uaggacgggg ggcggaaa 18
<210> 21
   <211> 10
   <212> PRT
   <213> Artificial
<220>
   <223> p1
<220>
   <221> MUTAGEN
   <222> (1)..(1)
   <223>
<220>
   <221> MISC_FEATURE
   <222> (1)..(1)
   <223> X= ClAB-L-Phe
<400> 21
   Xaa Thr Tyr Asn Glu Arg Leu Phe Trp Cys
   1               5                   10
<210> 22
   <211> 12
   <212> PRT
   <213> Artificial
<220>
   <223> p2
<220>
   <221> MUTAGEN
   <222> (1)..(1)
   <223>
<220>
   <221> MISC_FEATURE
   <222> (1)..(1)
   <223> X= ClAB-L-Phe
<400> 22
   Xaa Ser Gln Trp Asp Pro Trp Ala Ile Phe Trp Cys
   1               5                   10
<210> 23
   <211> 10
   <212> PRT
   <213> Artificial
<220>
   <223> p3
<220>
   <221> MUTAGEN
   <222> (1)..(1)
   <223>
<220>
   <221> MISC_FEATURE
   <222> (1)..(1)
   <223> X= ClAB-L-Phe
<400> 23
   Xaa Ser Phe Leu Glu Ile Gln Phe Trp Cys
   1               5                   10
<210> 24
   <211> 10
   <212> PRT
   <213> Artificial
<220>
   <223> p4
<220>
   <221> MUTAGEN
   <222> (1)..(1)
   <223>
<220>
   <221> MISC_FEATURE
   <222> (1)..(1)
   <223> X= ClAB-L-Phe
<400> 24
   Xaa Ser Phe Leu Asp Ile Gln Phe Trp Cys
   1               5                   10
<210> 25
   <211> 11
   <212> PRT
   <213> Artificial
<220>
   <223> p5
<220>
   <221> MUTAGEN
   <222> (1)..(1)
   <223>
<220>
   <221> MISC_FEATURE
   <222> (1)..(1)
   <223> X= ClAB-L-Phe
<400> 25
   Xaa Ser Tyr Leu Glu Arg Leu Phe Trp Cys Cys
   1               5                   10
<210> 26
   <211> 10
   <212> PRT
   <213> Artificial
<220>
   <223> p6
<220>
   <221> MUTAGEN
   <222> (1)..(1)
   <223>
<220>
   <221> MISC_FEATURE
   <222> (1)..(1)
   <223> X= ClAB-L-Phe
<400> 26
   Xaa Thr Tyr Asn Glu Leu Leu Phe Trp Cys
   1               5                   10
<210> 27
   <211> 12
   <212> PRT
   <213> Artificial
<220>
   <223> p7
<220>
   <221> MUTAGEN
   <222> (1)..(1)
   <223>
<220>
   <221> MISC_FEATURE
   <222> (1)..(1)
   <223> X= ClAB-L-Phe
<400> 27
   Xaa Ser Gln Trp Asp Pro Trp Ala Ile Phe Phe Gly
   1               5                   10
<210> 28
   <211> 12
   <212> PRT
   <213> Artificial
<220>
   <223> p8
<220>
   <221> MUTAGEN
   <222> (1)..(1)
   <223>
<220>
   <221> MISC_FEATURE
   <222> (1)..(1)
   <223> X= ClAB-L-Phe
<400> 28
   Xaa Ser Gln Trp Asp Pro Leu Gly Tyr Phe Leu Val
   1               5                   10
<210> 29
   <211> 13
   <212> PRT
   <213> Artificial
<220>
   <223> p9
<220>
   <221> MUTAGEN
   <222> (1)..(1)
   <223>
<220>
   <221> MISC_FEATURE
   <222> (1)..(1)
   <223> X= ClAB-L-Phe
<400> 29
   Xaa Glu Leu Asp Leu Ile Gly Leu Arg Ser Trp Phe Cys
   1               5                   10
<210> 30
   <211> 10
   <212> PRT
   <213> Artificial
<220>
   <223> Reversed p1
<220>
   <221> MUTAGEN
   <222> (1)..(1)
   <223>
<220>
   <221> MISC_FEATURE
   <222> (1)..(1)
   <223> X= ClAB-L-Phe
<400> 30
   Xaa Trp Phe Leu Arg Glu Asn Tyr Thr Cys
   1               5                   10
<210> 31
   <211> 10
   <212> PRT
   <213> Artificial
<220>
   <223> Shuffled p1
<220>
   <221> MUTAGEN
   <222> (1)..(1)
   <223>
<220>
   <221> MISC_FEATURE
   <222> (1)..(1)
   <223> X= ClAB-L-Phe
<400> 31
   Xaa Arg Phe Glu Thr Leu Asn Trp Tyr Cys
   1               5                   10
<210> 32
   <211> 12
   <212> PRT
   <213> Artificial
<220>
   <223> Reversed p2
<220>
   <221> MUTAGEN
   <222> (1)..(1)
   <223>
<220>
   <221> MISC_FEATURE
   <222> (1)..(1)
   <223> X= ClAB-L-Phe
<400> 32
   Xaa Trp Phe Ile Ala Trp Pro Asp Trp Gln Ser Cys
   1               5                   10
<210> 33
   <211> 12
   <212> PRT
   <213> Artificial
<220>
   <223> Shuffled p2
<220>
   <221> MUTAGEN
   <222> (1)..(1)
   <223>
<220>
   <221> MISC_FEATURE
   <222> (1)..(1)
   <223> X= ClAB-L-Phe
<400> 33
   Xaa Asp Gln Ser Pro Trp Trp Trp Ile Ala Phe Cys
   1               5                   10
<210> 34
   <211> 52
   <212> DNA
   <213> Artificial
<220>
   <223> 5'-SD-AUG (Ex.5)
<400> 34
   ctagtaatac gactcactat agggttaact ttaagaagga gatatacata tg 52
<210> 35
   <211> 44
   <212> DNA
   <213> Artificial
<220>
   <223> AUG-spacer-an21 (Ex.5)
<400> 35
   atgggaggtg gttcaggaag ttaggacggg gggcgggagg cggg 44
<210> 36
   <211> 7
   <212> PRT
   <213> Artificial
<220>
   <223> spacer (Ex.5)
<400> 36
   Trp Gly Gly Gly Ser Gly Ser
   1               5

## Description

### TECHNICAL FIELD

The present invention relates to an improved method which uses a display method for associating genotypes and phenotypes for creating linkages of mRNAs and translation products thereof, and the method is used for selecting functional proteins, peptides, and peptide analogs.

### BACKGROUND ART

Methods based on evolutionary molecular engineering, such as the phage display method and the mRNA display method, are used to create various functional proteins and peptides. In particular, the mRNA display method ("In vitro virus," Nemoto N, et al. FEBS Lett. 414, 405-408 (1997), WO 98/16636; or "RNA-peptide fusions," Roberts, R. W. & Szostak, J. W., Proc. Natl. Acad. Sci. USA., 94, 12297-12302 (1997), WO 98/31700) is a useful method, since it can generate peptide libraries with a high variety of about 10¹²⁻¹³.

However, there is a serious drawback to the mRNA display method. In this method, the peptide library can only be produced through many burdensome steps.

In the mRNA display method, the "one-to-one correspondence" between the amino acid sequence of a peptide and the base sequence of an mRNA is maintained by a covalent bond formed through a linker DNA. The formation of this peptide/linker DNA/mRNA complex requires (1) forming a covalent bond between a linker DNA and an mRNA by methods like enzyme reaction or photocrosslinking and (2) adding this combination to the cell-free translation system to link the mRNA and the translation product through puromycin on the end of the linker DNA.

The object of the above operation (1) is to link puromycin (Pu), which is an analog on the tyrosyl tRNA 3'-end, to the 3'-end of mRNA through a linker DNA before adding mRNA to the translation system. When the Pu-linker DNA/mRNA complex is introduced into the cell-free translation system in the synthesis of peptide from mRNA, puromycin attacks the C-terminus of the peptide chain in the ribosome while the chain elongates to form a substrate of the transpeptidation reaction, and the peptide molecule, which is a translation product, links with mRNA through puromycin.

Methods that are known for binding linker DNA and mRNA are ligation, photocrosslinking, or hybridization using 2'-0-methyl-RNA (Non-patent Documents 1-4). As seen, the reaction to attach a linker to an mRNA is performed as an independent step outside of the cell-free translation system. Hence, the mRNA display method requires complicated operations, such as performing transcription on DNAs in the pool, purifying the resulting mRNAs and connecting them with linkers, and further purifying the result to add to the translation system.

Accordingly, the mRNA display method is an extremely complex method requiring one to two days per one round of selection. Furthermore, an actual creation of peptides requires 5 to 10 rounds of selection to be performed, and if no useful peptide is obtained, the operation must be repeated again after changing the selection condition. Hence, a selection of useful peptides using an mRNA display method is time consuming, and a more rapid selection method waits to be developed.

### CITATION LIST

### PATENT DOCUMENTS

Patent Document 1: Japanese Patent No. 3683282 (WO 98/16636)
Patent Document 2: Japanese Patent No. 3683902
Patent Document 3: Japanese Patent No. 3692542 (WO 98/31700)

### NON-PATENT DOCUMENTS

Non-Patent Document 1: Nemoto N, et al. FEBS Lett. 414, 405-408 (1997)
Non-Patent Document 2: Roberts, R. W. & Szostak, J. W. , Proc. Natl. Acad. Sci. USA., 94, 12297-12302 (1997)
Non-Patent Document 3: M Kurz, K Gu, and P. A. Lohse, Nucleic Acids Res. 28 (e83)(2000)
Non-Patent Document 4: I. Tabuchi, S. Soramoto, N. Nemoto et al., FEBS Lett. 508 (3), 309 (2001)

### SUMMARY OF THE INVENTION

### PROBLEM TO BE SOLVED BY THE INVENTION

The present invention provides a rapid and easy method for selecting the desired peptide, protein or peptide analog.

### MEANS FOR SOLVING THE PROBLEM

To expedite the repeated operation of display and selection mentioned above, the number of process steps per round that involve a person should be as small as possible. Hence, the present invention provides a new reaction system that enables a single step formation of a translation product/linker/mRNA complex.

In the reaction system of the present invention, a series of reactions, namely, transcription, association of an mRNA and a linker, translation on the ribosome and formation of a complex consisting of a translation product and a linker/mRNA proceed automatically. This allows a library of translation products to be formed in substantially a single step. Hence, the time for preparation of translation products displayed on the corresponding mRNA can be markedly reduced. Or else, a similar effect of a rapid and easy, single-step formation of a translation product/linker/mRNA complex can be obtained by adding an mRNA library and linkers in a reaction system consisting only of a translation system (not including any factors necessary for transcription) and performing similar reactions.

Further, in this new reaction system, it is possible to add the DNA library recovered from the preceding round and subjected to PCR in that state without purification, and using it as a template DNA to perform a series of reactions from transcription to formation of translation product/linker/mRNA complexes mentioned above. This markedly reduces the time required for the selection operation of useful translation products.

The gist of the present invention is described below.

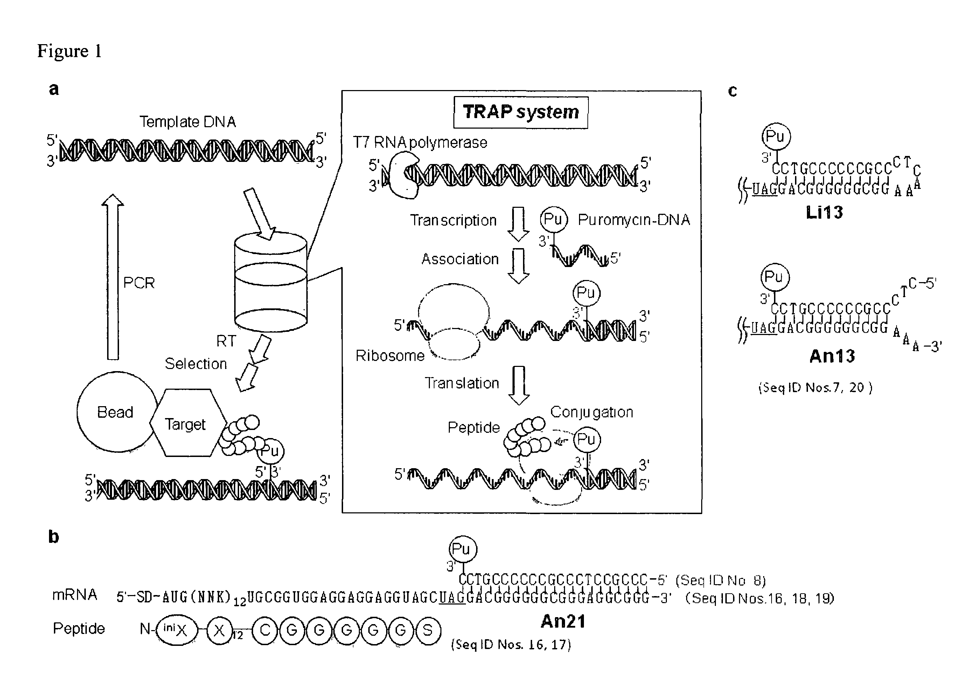

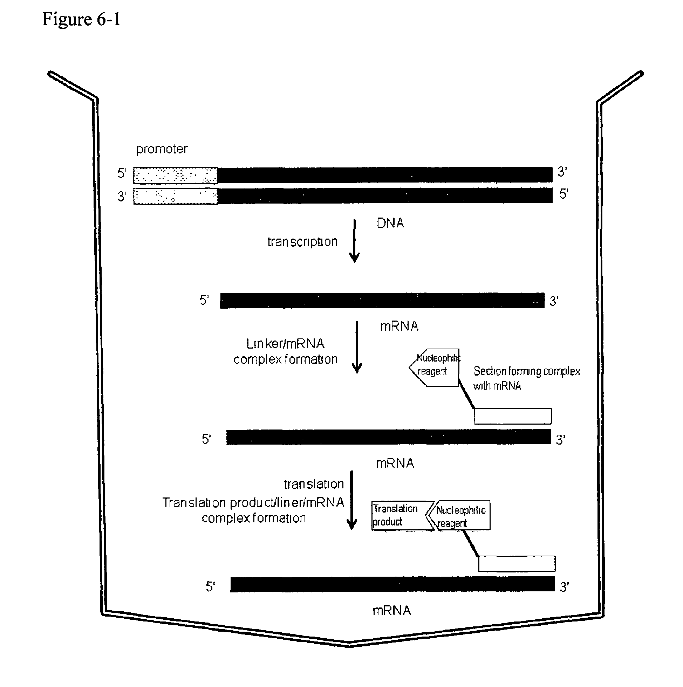

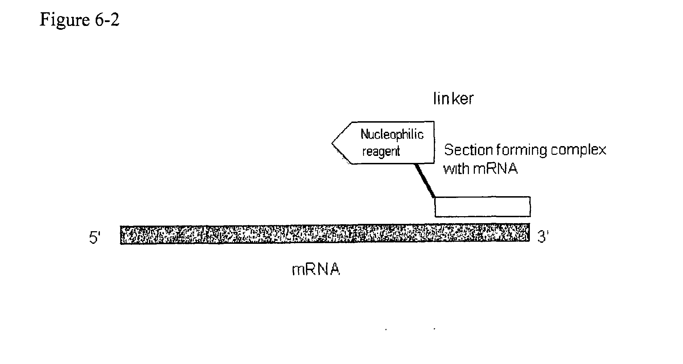

The particular transcription-linker association-translation coupling reaction system is shown in Fig. 6-1. Fig. 6-2 is a scheme of a linker.
(1) A transcription-linker association-translation coupling reaction system characterized by incorporation of a template DNA library to enable a step of forming translation product/linker/mRNA complexes through transcription of a template DNA library to mRNAs, association of mRNAs with linkers, translation of mRNAs, and binding with translation products to be automatically performed in a reaction system, comprising factors necessary for transcription, factors necessary for translation, and linkers.
   (1-2) The DNA contains a promoter sequence of transcriptase, and factors necessary for transcription in the reaction system enables rapid generation of the mRNA library from the template DNA library. It is preferable for the factors necessary for transcription to promote transcription from DNA to mRNA. For example, the reaction system may include transcriptase at 0.1 µM or higher.
   (1-3) It is preferable for the factors necessary for translation to promote translation. For example, the factors necessary for translation may include EF-P.
   (1-4) Factors necessary for transcription or factors necessary for translation in the cell-free transcription/translation system may be derived from an Escherichia coli extract, wheat germ extract, a rabbit reticulocyte extract, an insect cell extract, a human cell extract, etc.
   (1-5) The factors necessary for translation may include 20 types of aminoacyl tRNA synthetic enzymes (ARS).
   (1-6) It may be possible to introduce a codon with low translation efficiency or a blank codon that cannot be translated into the DNA sequence to stabilize translation product/linker/mRNA complexes in the translation system. The position of the codon may preferably overlap with the sequence where the linker anneals with mRNA or within 20 bases upstream of it.
   (1-7) The step of forming translation product/linker/mRNA complexes through transcription of a template DNA library to mRNAs, association of mRNAs with linkers, translation of mRNAs, and binding with translation products is completed in an hour.
(2) The mRNA and linker are bound in the translation system. The linkers each contain a section that forms a complex with an mRNA in a translation system, which includes nucleic acids such as DNA, RNA, and nucleic acid analogs, without being limited thereby. A base sequence of the linker in this section is annealed with a sequence of a 3'-end untranslated region of an mRNA, and an mRNA and a linker are bound through a covalent bond and/or a noncovalent bond.
   (2-2) It is preferable for the linker and each mRNA to be bound by a bond that is not a covalent bond.
   (2-3) The complementarity of first 10 bases on a 3'-end of a base sequence of a linker in the section that forms a complex with an mRNA and a sequence of a 3'-end untranslated region of an mRNA is preferably 50% or higher.
   (2-4) The first 13 bases on the 3'-end of the base sequence of the linker in the section that forms a complex with an mRNA may have the same base sequence as the Pu-linker of the An13-type in Fig. 1c.
   (2-5) The first 21 bases on the 3'-end of the base sequence of the linker in the section that forms a complex with an mRNA may have the same base sequence as the Pu-linker of the An21-type in Fig. 1b.
(3) Linkers include a nucleophilic reagent (nucleophile) that can be bound to a translation product, which include aminoacyl RNA, aminoacyl RNA analog, amino acid, amino acid analog, puromycin, puromycin analog, etc. that can be bound to a translation product, without being limited thereby.
(4) When using mRNA instead of DNA, the mRNA and linker may be bound by the above method, and reaction systems consisting solely of a translation system may also be used.
(5) A method of forming translation product/linker/mRNA complexes in a single step to create a translation product library. The step includes incorporating a template DNA library in the transcription-linker association-translation coupling reaction system, and the reaction system includes factors necessary for transcription, factors necessary for translation, and linkers described in (1) to (4).
(6) A nucleic acid library that encodes translation products having desired functions may be created by selecting a translation product having a desired function from the library of translation products created by the above method, and amplifying an mRNA or a cDNA binding with a selected translation product. The nucleic acid library is incorporated to the reaction system again as a template to obtain a translation product library, and the selection/amplification step is repeated multiple times to obtain a nucleic acid library in which nucleic acids encoding useful translation products are condensed.
   (6-2) It is preferable to use the above method in the second round or subsequent rounds. In such case, the PCR product in its original state without purification may be added as the template DNA library.
   (6-3) The translation product having the desired function may be a translation product binding to the target.
   (6-4) The above selection-amplification operation can be completed within 3 hours.
   (6-5) The above selection-amplification operation can be repeated twice or more per day.
   (6-6) The above selection-amplification operation can be automated by machine operation.
   (6-7) Targets include a purified target, a target inserted into the membrane, a target displayed on phage, a target displayed on baculovirus, a target displayed on the cell, without being limited thereby.
   (6-8) The translation product may be a peptide, protein, peptide analog, without being limited thereby.
   (6-9) The translation product may be modified by post-translation modification after the complex has been formed.
   (6-10) The method of (6-3) in which the target substance is human serum albumin.
(7) Compounds p1 and p2 of Example 4 obtained by the method of (6-10).

### ADVANTAGEOUS EFFECT OF THE INVENTION

In the present invention, a stable complex of a translation product and an mRNA is formed just by adding template DNAs to the cell-free translation system, which allows the selection operation that took one to two days in the conventional mRNA display method to end in about three hours.

Further, by introducing tRNA that supports non-proteinous amino acid or hydroxy acid (referred to collectively as "non-standard amino acid") into the translation solution, it is possible to obtain a non-standard peptide/linker/mRNA complex that displays non-standard peptide synthesized by translation from the sequence information in the template nucleic acid molecule.

For example, in a selection of a non-natural cyclic peptide that binds to human serum albumin using the present method, multiple non-natural cyclic peptides that have strong affinity were selected through six rounds of selections in 14 hrs. Cyclic peptides containing a non-proteinous amino acid (non-natural cyclic peptide) in the backbone are not readily decomposed by peptidase, so they are seen with expectation as candidates of pharmaceutical agents and bioprobes.

Accordingly, since the present invention allows a rapid selection of a non-natural cyclic peptide binding to the target, it should speed up the creation of a non-natural cyclic peptide as a pharmaceutical agent candidate or a bioprobe. In addition, the present method can be used for peptides and proteins consisting of proteinous amino acids, so it is expected to enable a rapid creation of functional peptides and antibodies.

### BRIEF DESCRIPTION OF THE DRAWINGS

Figure 1 shows a selection operation using a reaction system of Transcription-translation coupled with Association of Puromycin-linker (TRAP system). (a) A scheme of the selection operation consisting of the four steps of formation of a translation product/linker/mRNA complex in the TRAP system, reverse transcription (RT), selection of the target-immobilized beads, and PCR. (b) A complex of a random pool of mRNA and Pu-linker (An21) used in the Examples. (c) An mRNA/Pu-linker structure of a 13-base-pair duplex with ligation (Li13) or without ligation (An13).
Figure 2 shows a study of the stability of peptide/linker/mRNA complex using T7-peptide pull-down assay. (a) A T7-peptide pull-down assay scheme. The T7-peptide template and the random pool (NNK)₁₂ template were translated, and subjected to reverse transcription (RT), and then the complex was recovered by anti-T7 antibody-immobilized beads. (b) Assessment of the peptide/linker/mRNA complex stability.
Figure 3 shows the efficiency of the random peptide/linker/mRNA complex formation in a TRAP system. (a) The biotin-L-Phe (^{Bio}F) structure used in the synthesis of biotin-label peptides. (b) Scheme of the biotin pull-down assay. The N terminuses of synthesized peptides were labeled with biotin using ^{Bio}F-tRNA^{fMet}(CAU). The biotinized peptide/linker/mRNA/cDNA complexes were captured by streptavidin magnetic beads after translation and reverse transcription. The recovered complexes were quantified by real-time PCR. (c) Efficiency of forming random peptide/mRNA complex.
Figure 4 is comparison of enrichment of the T7-peptide template between conventional mRNA display (Li13) and a method using an An21 type linker.

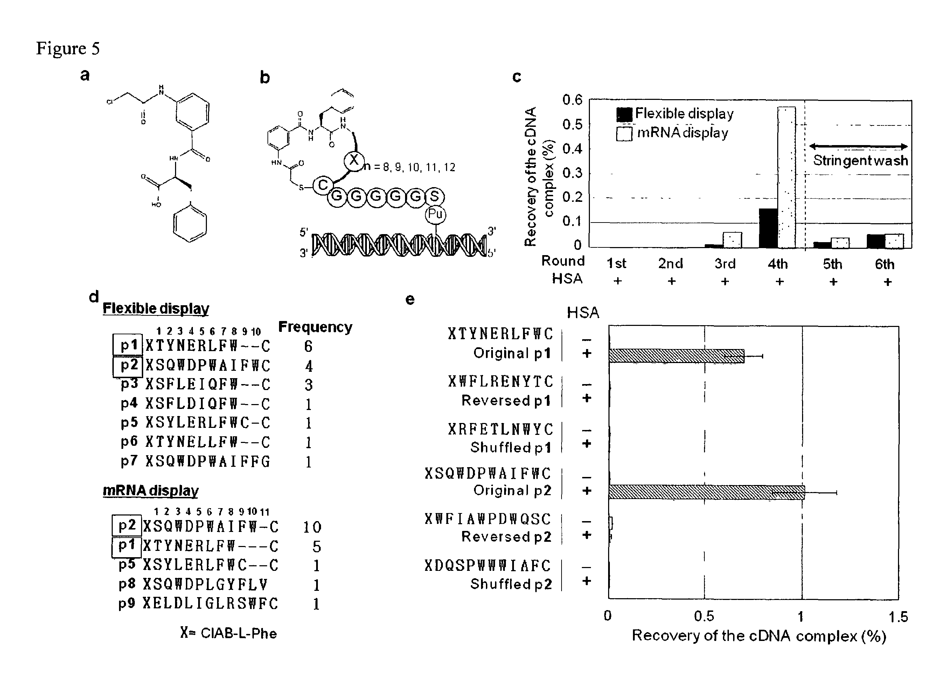

Figure 5 is an *in vitro* selection of HSA binding cyclic peptide using the Flexible Display method and the mRNA Display method. (a) A structure of N-[3-(2-2-chloroacetamido)benzoyl]-L-phenylalanine (ClAB-L-Phe) used for peptide cyclization. (b) A cyclic peptide library used in Example 4. A random mRNA/Pu-linker complex displaying a cyclic peptide consisting of random amino acid sequence (n=8-12) having ClAB-L-Phe and Cys at both ends. (c) Process of Flexible Display selection and mRNA Display selection using the HSA-immobilized SA-beads. (d) Sequence of selected peptides. X and C respectively indicate ClAB-L-Phe and Cys. (e) Assessment of the selected peptide.
Figure 6-1 is a scheme of the reaction that proceeds by the cell-free transcription-translation reaction system containing a linker.
Figure 6-2 is a scheme of a linker.
Figure 7 is progress of mRNA transcription in the TRAP system. The amounts of the transcription product, mRNA, at the respective timings were measured by RT real-time PCR. The transcription-translation coupled reaction solution (1 µL) was performed at 37°C for 20 min. The random-mRNA template (1 µM) in the translation solution was used as the standard. The error bar shows the standard deviation obtained by three rounds of experiment.
Figure 8 is the binding curve of the peptides, p1 (top) and p2 (bottom), to HSA was obtained by the BLI method.

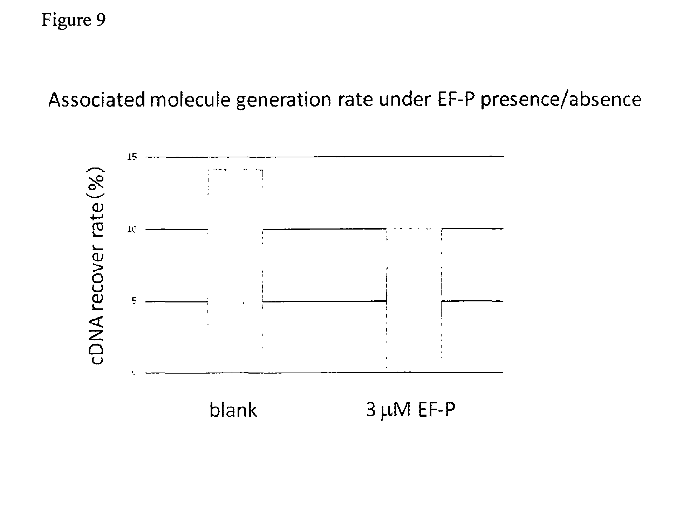

Figure 9 is the generation of association molecules under the presence/absence of EF-P was confirmed.

### BEST MODE FOR CARRYING OUT THE INVENTION

### 1. Transcription-Linker Association-Translation Coupling System

The first aspect of the present invention is a cell-free transcription-translation coupling system containing a linker. The reaction system includes factors necessary for transcription/translation, and linkers having an acceptor for a translation product, and through adding a template DNA library to the reaction system, it enables the formation of a complex consisting of a translation product/linker/mRNA to proceed automatically through transcription of the template library to mRNA, association of mRNAs and linkers, translation of mRNA on the ribosome, and linkages with the translation product.

Since the step of forming a translation product/linker/mRNA complex is performed automatically in the reaction system, no additional work is necessary. In other words, according to the reaction system of the present invention, the only work required for the step of forming a translation product/linker/mRNA complex is to add the template DNA library to the reaction system, and the force of the components in the reaction system promotes transcription, formation of a complex consisting of a linker and mRNA, and formation of a complex consisting of the translation product, the linker and mRNA. The reaction that proceeds by the reaction system of the present invention is shown schematically in Fig. 6.

For further detailed explanation, an example of the first aspect of the present invention is shown by the "TRAP system" on the right of Fig. 1a. It shows a T7 RNA polymerase for transcription and a linker whose acceptor for the translation product is puromycin (Pu). However, this is merely an example and the present invention is not limited to the embodiment represented by Fig. 1a. The transcription-linker association-translation coupling system of the present invention will be referred to hereinafter as the TRAP system. As a variation of the TRAP system, it is possible to use mRNA that has been preliminarily transcribed (linker association-translation coupling system), and in such a case, factors necessary for transcription do not need to be added. However, the term, cell-free translation system, refers to the reaction system containing factors necessary for transcription (transcription/translation system). Hence, the terms cell-free translation system and the cell-free transcription/translation system are used as interchangeable terms.

Figure 1a is a scheme of a selection operation consisting of the four steps of forming a translation product/linker/mRNA complex in the TRAP system, reverse transcription (RT), selection of the target-immobilized beads, and PCR. The reaction in the TRAP system illustrated in the balloon on the right side of Fig. 1a, is a single step in which transcription, association of Puromycin-DNA linkers, translation, and formation of a complex of the translation product (Peptide) and the peptide acceptor are coupled.

In the TRAP system, it is possible to use an optional cell-free translation system. The system generally includes a ribosome protein, a ribosome RNA, an amino acid, tRNA, GTP, ATP, initiation and elongation factor, and other factors necessary for translation. The publicly known cell-free translation system that can be used in the TRAP system includes an Escherichia coli extract, wheat germ extract, a rabbit reticulocyte extract, an insect cell extract, an animal cell extract. The cell-free translation system is a system in a test tube that does not use a live living organism, so it is possible to arbitrarily change various conditions such as the composition of the reaction solution and the reaction temperature. Modified systems are preferably used by means such as removing certain components or adding necessary components. Or else, it is possible to use a translation system that has been reconstructed by combining components necessary for transcription and/or translation that have been isolated from one or more arbitrary organisms Or else, it is possible to appropriately combine a component that has been synthesized in vitro to a component that has been isolated from one or more arbitrary organisms. The isolated components can be separated/purified from an organism source, but they can also be produced by a genetic engineering method or chemical synthesis, or a method that combines these methods. The explanation in the present specification and the Examples provided below use a system derived from prokaryote, but that is only for the sake of explanation, and there is no intent to exclude using a reaction system that uses eukaryote as the source of transcription/translation enzymes. For example, a transcription/translation system derived from plant seeds can be preferably used in the TRAP system. In particular, a system derived from wheat germ extracts is a publicly known, highly efficient system, which is preferable.

The difference between the conventional cell-free translation system and the TRAP system is that the latter includes the linker as a mandatory component.

To complete the present invention, the present inventors compared the stability of translation product/linker/mRNA and the efficiency of translation product library formation when using the TRAP system and when using a linker for a publicly known mRNA Display method in a conventional cell-free translation system.

The cell-free translation system using a component derived from a purified Escherichia coli was used as the conventional cell-free translation system in the Examples. This is a translation system that separates and purifies factors relating to the translation reaction such as the ribosome of Escherichia coli, transfer RNA (tRNA), and aminoacyl tRNA synthetic enzyme (ARS), then optionally mixes the factors for reconstitution. The technologies in the following documents are publicly known, for example: H. F. Kung, B. Redfield, B. V. Treadwell, B. Eskin, C. Spears and H. Weissbach (1977) "DNA-directed in vitro synthesis of beta-galactosidase. Studies with purified factors" The Journal of Biological Chemistry Vol. 252, No. 19, 6889-6894; M. C. Gonza, C. Cunningham and R. M. Green (1985) "Isolation and point of action of a factor from Escherichia coli required to reconstruct translation" Proceeding of National Academy of Sciences of the United States of America Vol. 82, 1648-1652; M. Y. Pavlov and M. Ehrenberg (1996) "Rate of translation of natural mRNAs in an optimized in Vitro system" Archives of Biochemistry and Biophysics Vol. 328, No. 1, 9-16; Y. Shimizu, A. Inoue, Y. Tomari, T. Suzuki, T. Yokogawa, K. Nishikawa and T. Ueda (2001) "Cell-free translation reconstituted with purified components" Nature Biotechnology Vol. 19, No. 8, 751-755;H. Ohashi, Y. Shimizu, B. W. Ying, and T. Ueda (2007) "Efficient protein selection based on ribosome display system with purified components "Biochemical and Biophysical Research Communications Vol. 352, No. 1, 270-276.

The typical components of a cell-free translation system using the components derived from Escherichia coli include the following: (i) T7 RNA polymerase (when also performing transcription from DNA), (ii) translation factors, such as initiation factors of Escherichia coli (e.g. IF1, IF2, IF3)/elongation factors (e.g. EF-Tu, EF-Ts, EF-G)/ release factor and ribosome recycling factor (e.g. RF1, RF2, RF3, RRF), (iii) 20 types of aminoacyl tRNA synthetic enzyme (ARS), methionyl tRNA (isolation from Escherichia coli), (vi) various amino acids, NTP, energy recycling system (e.g. creatine kinase, myokinase, pyrophosphatase, nucleotide-diphosphatase kinase and other enzymes for recycling energy source are included. A translation system that adds artificially synthesized aminoacyl tRNA is also publicly known, and used to introduce mainly non-proteinous amino acid and hydroxy acid to the translation product (A. C. Forster, Z. Tan, M. N. L. Nalam et al., "Programming peptidomimetic syntheses by translating genetic codes designed de novo," PNAS 2003, vol. 100, no. 11, 6353-6357;T. Kawakami and H. Murakami "Genetically Encoded Libraries of Nonstandard Peptides," Journal of Nucleic Acids Volume 2012 (2012), Article ID 713510, 15 pages_).

The TRAP system of the present invention (including variations thereof) is characterized in that it incorporates linkers having an acceptor to the translation product, in addition to factors relating to the translation reaction. This characteristic leads to all reactions consisting of association of mRNA and the linker, the translation of mRNA on the ribosome, and the conjugation of mRNA and the translation product through the linker to occur in the cell-free translation system. Hence, the linker molecule in the TRAP system has a structure for forming a complex with mRNA in the cell-free translation system. In contrast, the binding of mRNA and the linker for a linker in the publicly known mRNA Display method is brought about by a linking reaction outside the translation system. The structure of the linker used in the TRAP system is described in detail hereinafter.

Further, the components of the reaction system are optimized by modification from a publicly known cell-free translation system in the TRAP system. An example of the modified cell-free translation system is a translation system that does not include an amino acid, ARS, MTF, release factor, tRNA, etc.

For example, the TRAP system in the Examples does not include at least one release factor. A release factor is a proteinous factor necessary for recognizing the termination/initiation codons on the mRNA and freeing the completed peptide chain from a ribosome. By not having a release factor, translation can be temporarily suppressed without having to terminate it. The release factor is also called a peptide chain releasing factor, and it is classified into the two classes of Class I and Class II. The Class I releasing factor is thought to advance the peptide chain dissociation reaction by recognizing the termination codon and activating the peptide chain transition reaction center of ribosome. In the Escherichia coli, two types of Class I releasing factor named RF1 and RF2 are known, of which RF1 recognizes UAA and UAG, and RF2 recognizes UAA and UGA. The Class II dissociation factor is thought to promote the removal of the Class I releasing factor remaining on the mRNA in the ribosome after the peptide chain dissociation reaction by the Class I releasing factor. An example of the translation system available in the TRAP system lacks a Class I releasing factor. A system using the ribosome of Escherichia coli may lack both RF1 and RF2, or it may lack one of the two Class I releasing factors.

To improve the efficiency of the translation product library formation, it is preferable for the factors required for translation to promote the translation reaction on the ribosome.

For example, the factors required for translation may further include EF-P. EF-P is an elongation factor P of bacteria that is preserved at a high level, independent of the type. EF-P binds with ribosome, optimizes the interaction between tRNA and the dissociation factor to promote translation. The binding site of EF-P on the ribosome is positioned between the P site and the E site. The N terminus domain of EF-P interacts with the acceptor stem of tRNA bonding to the P site to promote formation of peptide bonds, and it stabilizes peptidyl tRNA in the catalyst center of peptidyl transferase of the ribosome. EF-P may be a modified EF-P protein, and it is preferable for the affinity of EF-P to ribosome to improve by modification. The EF-P modification may be in the form of post-translational modification (e.g. on the lysine residue).

Further, it is preferable in the TRAP system for the factors required for translation existing in the reaction system to promote the incorporation of non-standard amino acids into the translation product.

For this purpose, the TRAP system may be a system that is given limited amino acids. For example, a translation system that does not include at least one proteinous amino acid may translate/synthesize peptides that include non-standard amino acids on the ribosome based on the genetic information of mRNA by linking the codon of a proteinous amino acid that has not been added to an anticodon of tRNA that is acylated with non-standard amino acid. Or else, it is possible to translate/synthesize non-standard peptides that do not include any proteinous amino acid by adding acylated tRNA that has been priory bound to a non-standard amino acid to a system that does not contain any proteinous amino acid. It is also possible to add tRNA acylated with an arbitrary amino acid independent of whether it is a proteinous amino acid or not. The tRNA acylated by an arbitrary amino acid outside the system may be an orthogonal tRNA that is orthogonal to ARS existing in the translation system. An orthogonal tRNA is a tRNA that is not aminoacylated in the translation system since it is not recognized by ARS of a natural origin (e.g. an ARS protein enzyme of an Escherichia coli origin) inherent in the translation system, but that can express identified amino acid by efficiently pairing with mRNA codon in a peptide synthesis reaction on the ribosome. Examples of tRNA used as orthogonal tRNA include a natural suppressor tRNA derived from a different species or an artificially constructed tRNA. An acylated tRNA may be replaced by adding tRNA, ARS and a substrate thereof in the reaction solution. The TRAP system may contain 20 types of ARS.

If transcription is performed from template DNA, the TRAP system includes factors necessary to perform transcription from template DNA. Examples of factors required for transcription are suitable DNA dependent RNA synthetase or transcriptase and a substrate thereof, such as T7 RNA polymerase, T3 RNA polymerase, SP6 RNA polymerase. Each DNA in the template DNA library includes a promoter sequence corresponding to the transcriptase in the reaction system, and mRNA is transcribed. Factors required for transcription further include reduction agents such as nucleoside triphosphate (NTP), divalent magnesium ion, and DTT. In addition, polyamine such as spermidine, and a nonionic surfactant, Triton X-100, may be added as a reagent to assist stabilization or activation of polymerase.

As shown above, the present invention provides an improved method used in selecting a functional protein, peptide, and peptide analog from a random DNA pool which is the template of translation products, and the method repeats rounds consisting of the four steps of a TRAP system, reverse transcription (RT), selection and PCR. It has already been mentioned that a translation product/linker/mRNA complex (display library) is automatically formed in the reaction system by adding a template DNA library in the TRAP system. Further, the TRAP system allows DNA amplified in the previous round of PCR to be added in the original state without purification as a template DNA library by adjusting the factors required for transcription. This eliminates the need of a template DNA library required in the conventional mRNA Display method, and speeds up the process of generating an mRNA library from the template DNA library.

Accordingly, it is preferable for the factors required in transcription existing in the TRAP system to promote transcription from DNA to mRNA. In particular, factors required for transcription should preferably exist in amounts that enable a speedy generation of the mRNA library from the template DNA library. For example, factors required for transcription may include transcriptase of 0.1 µM or higher. In addition, the yield of translation product/linker/mRNA complex may be increased by raising the transcriptase concentration, and the transcriptase can be included in an amount of about 0.2 µM or higher, or 0.3 µM or higher, or 0.4 µM or higher, or 0.5 µM or higher, or 0.6 µM or higher, or 0.7 µM or higher, or 0.8 µM or higher, or 0.9 µM or higher. When using T7 RNA polymerase, it may be included in an amount of 0. 05 µM-20 µM, preferably 0.1 µM-20 µM, or 0.2 µM-10 µM, more preferably about 0.5 µM-about 5 µM, and even more preferably about 0.8- about 1.2 µM, particularly preferably about 0.9 µM-1.1 µM, or about 1.0 µM.

Note that "about" used with numerical values show all numerical values in (the greater one of) the experimental error of the value (e.g. within a 95% confidence interval of the average) or a range within 10% of the value.

### 2. Formation of Translation Product-Linker/mRNA Complex

The cell-free translation system (TRAP system) of the present invention is a reaction system containing linkers, and complexes of the linkers and mRNA are formed in the reaction system.

An example of a reaction that takes place in the TRAP system is explained below by referring to Fig. 1a. Firstly, the template DNA library is transcribed to mRNA. Then, mRNA is caught by a linker (shown as a Puromycin-DNA linker in the figure) through hybridization. Since a GC rich sequence complementing the linker DNA is positioned on the 3'-non-translation region (3'-UTR) of mRNA, a stable duplex is formed between the linker DNA molecule and the mRNA molecule through annealing of the base sequences. The ribosome moves along the mRNA chain from the initiation codon to the end of the open reading frame (ORF), and stalls immediately before the linker/mRNA duplex section. Then, the peptide acceptor on the linker (puromycin "Pu" in the drawing) attacks the nascent polypeptide chain (specifically, the binding site of peptide and tRNA in the peptidyl tRNA) to form a peptide-linker/mRNA complex. The peptide-linker/mRNA complex is stably retained even after the peptide chain is dissociated from ribosome.

Figs. 1b and 1c show the structure of a linker/mRNA complex. Fig. 1b shows a linker available in the TRAP system forming a complex with the mRNA random pool. It shows a structure in which the DNA base sequence section of the linker is annealed with 3'-UTR of mRNA to form a DNA/RNA duplex of 21 base pairs. In this example (An21), mRNA has a base sequence corresponding to a cysteine residue and a spacer (Gly-Gly-Gly-Gly-Gly-Ser) arranged downstream of the (NNK)₁₂ random sequence, and the GC rich sequence of 21 bases including the last G is an annealing sequence with the Pu-linker. N in the NNK codon is a ribonucleotide of A, U, C or G, and K is a ribonucleotide of U or G. In comparison, An 13 of Fig. 1c shows a structure of the Pu-linker/mRNA complex composed of a 13-base-pair duplex. Li 13 in Fig. 1c is an example of a linker/mRNA linkage structure using a linker of a publicly known mRNA Display method, which constitutes a comparative example of the present invention, and the 3'-end of mRNA is ligated and forms a covalent bond with the 5'-end of the Pu-linker.

The termination/initiation codon UAG is positioned at the end of the open reading frame of mRNA. In the translation system whose translation release factor RF1 has been excluded, the UAG codon is blank, and putting the UAG codon in place enhances the effect of stalling the ribosome before the linker/mRNA duplex section when terminating translation.

The duplex section formed in a hydrogen bond is dissociated relatively easily when it contacts ribosome having RNA helicase (duplex splitting enzyme) activity.

Accordingly, stalling ribosome on mRNA by a blank codon is significant in the following two points: (1) stabilizing the bond between the linker and mRNA to hinder the peptide-linker section from dissociating from mRNA in the peptide-linker/mRNA complex to maintain the correct one-to-one correspondence of the translation product and the template nucleic acid; and (2) delaying the attack on the nascent polypeptide chain by the peptide acceptor to improve the efficiency of peptide-linker/mRNA complex formation.

The blank codon can be created by methods other than removing RF1. Also, a blank codon can be replaced with a minor codon having low translation efficiency. Hence, it may be possible to introduce a codon with low translation efficiency or a blank codon that cannot be translated into the sequence of the template nucleic acid. The position of a codon with low translation efficiency or blank codon may preferably be in the sequence where the linker anneals with mRNA (3'-UTR annealing sequence) or within 20 bases upstream of it. It is possible to introduce a blank codon or a codon with low translation efficiency at the end of ORF as shown in Fig. 1b.

The blank codon is a codon that is not translated. It is possible to create a blank codon by removing the proteinous factor such as an amino acid or an amino acyl synthetase or tRNA or the release factor from the cell-free translation system. For example, it is possible to make the codon having a designated amino acid in the genetic code table "blank" by removing a specific proteinous amino acid and the ARS corresponding to the amino acid.

A minor codon is a codon with low codon usage, which differs by the type of organism. The minor codon may be investigated by a public database such as http://www.kazusa.or.jp/codon/. A suitable minor codon corresponding to the translation system to be used can be selected and used in the present invention. Researchers studying the frequency of codon use in genes expressed in Escherichia coli revealed many codons with low frequency of use. Examples of such codons include AGA/AGG/CGA (Arg), AUA (Ile), CUA (Leu) GGA (Gly), CGG (Arg), CCC(Pro) codon (Ikemura, T., (1981) J. Mol. Biol. 146, 1-21; Dong, H. et al., (1996) J. Mol. Biol. 260, 649-663).

However, blank codons and minor codons are not necessarily requisites in forming peptide-linker/mRNA complexes.

A linker links mRNA with its translation product by binding to the 3'-end of mRNA at one end and the C-terminus of peptide at the other. Such function of the linker is the same in a TRAP system or in a conventional mRNA display method when adding a linker/mRNA linkage to the cell-free translation system.

The mRNA and linker are bound through a covalent bond and/or a non-covalent bond. The mRNA preferably anneals with the linker at the 3'-end sequence (3'-UTR) outside the code region, and bonds with the linker through a covalent bond or a non-covalent bond. Accordingly, the mRNA has a predetermined near 3'-end sequence (3'-UTR annealing sequence), and the linker has a section that forms a complex with mRNA in the translation system. The linker structure of a section that forms a complex with mRNA is a structure having a side chain of the nucleic acid base, and they can be nucleic acids such as DNA, and RNA, or a nucleic acid analog, without being limited thereby.

In one embodiment, the 5'-end of the nucleic acid base section of a linker and the 3'-end of an mRNA molecule form a complex by hybridization based on base pair formation (that is, a hydrogen bond) without forming a covalent bond between a linker and each mRNA. Hence, the 5'-end of the linker and the near 3'-end sequence of mRNA are preferably sequences that can bind with each other over a few bases to a few dozen bases by forming base pairs. Sequences that can bind by forming base pairs are not limited to those that are completely paired (that is, 100% complementary), but desynaptic bases may also exist as long as they cause no functional problems. A higher compatibility between the linker base sequence and the 3'-UTR annealing sequence of the mRNA molecule leads to a higher hybridization efficiency, and a higher stability. Meanwhile, it is preferable for the compatibility of the first 10 bases on the 3'-end of the linker base sequence of the section forming a complex with mRNA and the sequence in the most upstream point of 3'UTR of mRNA (that is, the part that approximates the code region of the 3'-UTR annealing sequence) to be 50% or higher, but the more downstream sequences may not have any pairing base at all.

In the present specification, the section forming a duplex of the linker base sequence and the 3'-UTR annealing sequence of mRNA molecule by hybridization is referred to as a linker/mRNA duplex section.

In a different embodiment, the mRNA and linker bonds through covalent bond after annealing. For example, a covalent bond can be formed after annealing in the reaction system for a photocrosslink reaction, or by incorporating a non-natural crosslinking base. A structure of a base that enables such crosslinking reaction is publicly known.

As mentioned above, the other end of the linker has a structure that can bind to the translation product. Terms generally used in the conventional mRNA display method is used in the present invention to refer to this section as "peptidyl acceptor" and the translation product as "peptide" or "peptide chain," but the translation product actually synthesized on ribosome includes polymers other than those formed through a typical peptide bond.

A peptide acceptor is a molecule that can receive a peptide (peptidyl tRNA) C-terminus in the process of elongation by the peptide transition reaction on the ribosome, and that can form a covalent bond with peptide. Many molecules satisfying such conditions are publicly known, and arbitrary peptide acceptors can be used in the present invention.

Peptide acceptors include nucleophilic compounds that can bond with the translation product, such as aminoacyl RNA, aminoacyl RNA analog, amino acid, amino acid analog, puromycin, puromycin analog, without being limited thereby.

A bond between a typical peptide acceptor and a peptide C-terminus is formed when an amino group on the peptide acceptor incorporated into the A site approaches the ester bond on the C-terminus of a peptide attached to the peptidyl tRNA on the P site, similar to the standard peptide transfer reaction on the ribosome. Hence, an amide bond is typically formed as the covalent bond with the peptide chain C-terminus. A specific example of a peptide acceptor is puromycin (Pu), similar to the publicly known mRNA Display method. Compounds having various nucleophilic functional groups, such as a hydroxide group or a thiol group, in addition to the amino group, may be used for forming a covalent bond with the translation product. Arbitrary compounds that function as a peptide acceptor at the end of a linker are collectively referred to as "nucleophilic reagents" in the present specification.

Sections of the linker other than the two ends have the same structure as those of the linkers used for publicly known mRNA display methods. For example, oligonucleotides such as a single stranded or duplex DNA or RNA, polyalkylene such as polyethylene, polyalkylene glycol such as polyethylene glycol, polystyrene, straight chain materials such as polysaccharides or combinations thereof are appropriately used.

Specific non-limiting examples of linkers that can be used in the present invention include a Pu-DNA linker in which the part forming a complex with mRNA is a single stranded DNA, and the part connecting to the dCdC-Pu-3' sequence on the 3'-end is polyethylene glycol. A typical example is the linker used in the Examples.

In the present invention, template nucleic acids with the necessary sequences are supplied to the cell-free transcription-translation coupling system consisting of component factors optimized according to their purposes. The DNA sequences need to be those that allow transcription of cDNAs to mRNAs in the TRAP system. Such sequences include a promoter sequence that corresponds to the RNA polymerase to be used. For mRNA translation to be started, there needs to be a suitable sequence upstream of the initiation codon. Such sequence, in case of ribosome derived from Escherichia coli, includes a SD sequence.

The initiation codon is a codon that indicates the start of translation, and it encodes an initiation amino acid constituting the N terminus of the translation product on the mRNA. AUG which is the codon for methionine is generally used as the initiation codon, so the initiation tRNA holds an anticodon corresponding to methionine and the initiation tRNA carries methionine (formyl methionine in a prokaryotic cell). However, translation may be initiated by binding an arbitrary amino acid other than methionine to the initiation tRNA by utilizing genetic code reprogramming. Further, an alteration of the anticodon sequence of the initiation tRNA makes it possible to use sequences other than AUG as the initiation codon.

It is preferable for the code region to be arranged of a spacer sequence consisting of a peptide that provides flexibility linking to the C-terminus of the section encoding random amino acid sequences for the peptide library, immediately followed by a termination codon or a blank codon or a minor codon. For example, in the example of Fig. 1b, an amino acid sequence Gly-Gly-Gly-Gly-Gly-Ser is encoded after cysteine, immediately followed by the encoding of a blank codon UAG. When it is written, immediately following the spacer sequence, that expression does not mean that a sequence must be in contact with the spacer sequence, but instead, there may further be non-code sequences or other straight chain structures downstream of the spacer sequence. The position of a blank codon or a minor codon may be in the 3'-UTR annealing sequence of mRNA or further upstream.

As a principle, the UAG codon used as the termination codon can only be changed into a blank codon by removing the corresponding releasing factor from the cell-free translation system to change. For example, it is necessary to prepare the reaction solution by removing RF1 when using UAG (amber codon), RF2 when using UGA (opal codon) and both RF1 and RF2 when using UAA (ocher codon).

Further, as mentioned above, a sense codon may be turned into a blank codon by removing a specific proteinous amino acid and/or ARS corresponding to the amino acid.

Random sequences are composed of repetition of codons consisting of arbitrary base sequences. Examples of triplets on the mRNA sequence constituting a random sequence include a NNU codon or a NNK codon {N is a ribonucleotide of either A, U, C or G, and K is a ribonucleotide of either U or G}. The advantage of a NNU library is that a library with high certainty may be constructed since no stop codon defined by UAA, UAG and UGA appears. Codons that consist of four bases can be used as well as the standard three base (triplet) codons.

A sequence that can bind to the linker by forming a base pair (3'-UTR annealing sequence) is arranged at the 3'-end untranslated region (3'-UTR) of mRNA.

### 3. Selection-Amplification Operation

Useful translation products may be amplified by selecting a translation product that binds to the target from the library of "translation product-linker/mRNA complexes" created by the TRAP system of the present invention, and amplifying the mRNA or DNA binding to the translation product to further create a translation product library by the TRAP system. This is the second aspect of the present invention.

A publicly known method generally used in evolutionary molecular engineering may be used for selecting a translation product that binds to the target.

The evolutionary molecular engineering is aimed at creating proteins and peptides that have the desired function or characteristics, and clones having the target phenotype are selected from possible genes prepared in a large amount.

Basically, the DNA library is first prepared, then the RNA library is obtained as an in vitro transcription product, and the peptide library is obtained as the in vitro translation product. From the peptide library, targets with the desired function or characteristic are selected by some sort of a screening system. For example, if there is a need to obtain peptide molecules that bind to specific proteins, magnetic beads of solid phased target protein are mixed with the peptide library to recover a mixture of peptide molecules bound to the target protein using a magnet. Then, since each peptide molecule has an mRNA which is a template of the peptide attached to it like a tag, the mRNA in the library of recovered peptide-mRNA complexes is returned to DNA by reverse-transciptase, then amplified with PCR to obtain a biased library containing many clones with the targeted phenotype, to subsequently perform the same kind of selection experiment. Or else, it is possible to perform reverse transcription before selection to turn the nucleic acid section into a duplex, and thus avoid the possibility of recovering an RNA aptamer. By repeating this operation, clones having the desired phenotype will become enriched in the library with generation.

The first step in identifying peptide aptamers is to perform PCR to prepare a nucleic acid library from the nucleic acid section of the selected associated molecules and to determine the base sequence. Then, translation is performed according to the genetic code using the base sequence to obtain a sequence information of peptide aptamer that binds to the target substance.

It is convenient to modify the target substance in advance so that it can be recovered by binding to the solid phase to separate a complex of an active specie that binds to the target substance from other complexes. For example, in the Examples hereinafter, the target substance is modified in advance with biotin, and it is recovered using its ability to specifically bind to the biotin-binding protein forming a solid phase on the magnetic beads. Such specific bonds that can be used include not just the combination of a biotin binding protein (avidin, streptavidin)/biotin, but also maltose binding protein/maltose, polyhistidine peptide/metal ion (nickel, cobalt, etc.), glutathione-S-transferase/glutathione, antibody/antigen (epitope), without being limited thereby. Further, a non-specific absorption on the solid phase or the formation of a random covalent bond with a functional group on the solid phase can be used as a method for immobilizing the target substance.

The use of evolutionary molecule engineering allows peptide and protein having an amino acid sequence that does not naturally exist to be obtained as a gene library from DNA sequences of four randomly bonded bases, A, T, G and C. Further, tRNA that has been acylated with a non-proteinous amino acid (or hydroxyl acid) may be introduced into the translation system to synthesize by translation a non-standard peptide incorporating a non-proteinous amino acid as an in vitro translation product. Accordingly, the translation product may be a peptide, protein consisting of a proteinous amino acid, or a peptide analog that includes non-standard amino acid, without being limited thereby. The present invention enables a speedy creation of peptides, proteins, peptide analogs having useful functions by repeating the process of selecting active species displaying the desired bonding characteristics from a library of complexes of various translation products and mRNA (or cDNA) that can be subjected to template dependent synthesis, and amplifying the associated gene part, then translating again.

Suga et al. established a method called the Fit System (a method to translate/synthesize non-standard peptides using the reprogramming of genetic code) and the Rapid System as a method to search peptides having a physiological activity from a library of non-standard peptides using the conventional mRNA display method (Yuki Goto, Takayuki Katoh & Hiroaki Suga, Nature Protocols, 6, 779-790 (2011); Christopher J Hipolito and Hiroaki Suga, Current Opinion in Chemical Biology 2012, 16(1-2):196-203; Atsushi Yamaguchi, Takayuki Katoh & Hiroaki Suga, Drug Delivery System 26-6: 584-592, 2011). The flow of the Rapid system specifically exemplified by application to a non-standard peptide library containing thioether cyclic peptides is shown below (cited hereinafter from Jun Yamaguchi, Takayuki Katoh, Hiroaki Suga "Drug discovery of non-standard peptide with genetic code reprogramming" Drug Delivery System 26-6: 584-592, 2011).

"Firstly, a DNA library having a random sequence of amino acids (NNK sequence: the N on the 1^{st} and 2^{nd} bases are all of the bases of A, C, G and T, the K of the 3^{rd} base is the two base types of G, T) is prepared in the region sandwiched by an initiation codon (ATG) and a cysteine codon (TGT), and the library is transcribed to obtain an mRNA library constituting a template. Then, a linker consisting of puromycin added to the 3'-end of mRNA is ligated. Translation is performed by the FIT system using mRNA linked to a puromycin linker as a template in a translation system in which the initiation codon is reprogrammed with an amino acid modified with chloroacetyl group. After the translated/synthesized peptide and a template mRNA are linked through puromycin, a voluntary and irreversible cyclization of peptide occurs. A library of mRNA-non-standard peptide complexes can be constructed by the above process, in which the mRNA encoding the amino acid sequence information is linked with non-standard peptides. The mRNA section of the complex is reverse-transcribed and mixed with the target protein immobilized to the solid phase support, then complexes that were bound non-specifically or that were not bound were washed off to select only the non-standard peptide-mRNA-cDNA complex bonding to the target protein. Then, the cDNA of the recovered complex is amplified by PCR and transcribed again to be transformed to an mRNA library, then processes such as ligation and translation/synthesis are repeated to enrich peptides that bind to the target protein in the library, and the cDNA of the peptide complex that is finally obtained is recovered to analyze the base sequence. This enables analysis of amino acid sequences of specific non-standard peptides that bind to the target protein."

The selection operation using the Rapid system using the conventional mRNA display method and that using the transcription-linker association-translation coupling reaction system (TRAP system) of the present invention differ mainly in the following two points: (1) the former uses a linker binding to mRNA by ligation, but the latter uses a linker that captures mRNA by annealing; (2) by using the transcription-translation reaction system (TRAP system) containing such linker, the translation product/linker/mRNA complex is formed automatically just by incorporating a cDNA library to the reaction system.

Fig. 1a is a scheme of the selection operation brought about by the present invention. Firstly, a template DNA library (Template DNA) is created, then, the library is added to the TRAP system (Transcription → Association → Translation → Conjugation) to create a library of translation product/linker/mRNA complexes. Then, reverse transcription (RT) is performed to form translation product/linker/mRNA-cDNA complexes followed by selection. The cDNAs recovered from the thus obtained complexes are amplified (PCR) to be used as the template DNA library of the next round.

The amplified PCR reaction solution containing cDNA can be added to the TRAP system in its original state without purification. Hence, the selection operation using the TRAP system consists of the four steps of (1) forming translation product/linker/mRNA complex, (2) reverse transcription (RT), (3) selection, and (4) PCR as one round, and the rounds are repeated multiple times. In other words, step 1 can be performed immediately after step 4. On the other hand, the selection operation using the conventional mRNA display method requires additional steps in which cDNA after PCR is purified and subjected to a transcription reaction, then the result is purified and linked with linkers, and further, the result is purified and added to the translation system.

In the present invention, it is possible to use a linker association-translation coupling reaction system (variation of the TRAP system) to add previously transcribed mRNA in the first round, then use a transcription-linker association-translation coupling system (TRAP system) using the amplified DNA as the template in the second round and subsequent rounds.

In addition, it is also possible to use a translation system using mRNA as the template (a variation of the TRAP system) after the second round. In such a case, the mRNA is separately transcribed from cDNA in each round, then the transcription reaction solution in its original state without purification is added to the translation reaction system containing linkers. This method is useful when using mRNA that is difficult to transcribe.

In an actual creation of useful peptides and proteins, the above series of operations is typically repeated 5 or more times.

In the present invention, the selection operation that takes one to two days by the conventional mRNA Display method completes in about 3 hrs. In other words, it is possible to complete the series of operations (one round) in 3 hrs. Further, it is easy to perform two rounds in a day. For genes that can be easily transcribed, it is possible to complete a single round in 2.5 hrs or less.

For example, in the Examples described hereinafter, multiple non-natural cyclic peptides that bind strongly were selected after six times of selection in 14 hrs in a selection of non-natural cyclic peptides that bind to human serum albumin.

The above selection-amplification operation may be automated by machine operation.

The present invention also allows modification of the translation product by post-translation modification after forming complexes. The post-translation modification is a reaction that changes the chemical structure of the synthesized protein based on the genetic sequence, and is exemplified by phosphorylation, addition of sugar chain, addition of lipid, methylation, acetylation, etc. Modification by polypeptides such as ubiquitin and SUMO is also included in the post-translation modification.

### 4. Target Substance

The target substance may be an arbitrary compound that can be useful for interaction with the translation product. The target substance may be protein, nucleic acid, sugar, lipid or any other compound.

A preferable example of a target in the present invention is a substance that may become the target of pharmaceutical agent development.

Targets include a purified target (including partial purification), a target inserted into the membrane, a target displayed on phage, a target displayed on baculovirus, a target displayed on the cell, without being limited thereby.

An example of a specific target is serum albumin. Serum albumin is the most richly existing protein in serum, and it has many effects. For example, it acts as a carrier protein for numbers of hydrophobic molecules such as fatty acid and pharmaceutical agents, or it acts as the energy source for tumor cells (Elena Neumann, Eva Frei, Dorothee Funk et al., Expert Opin. Drug Deliv. 7 (8), 915 (2010)). Further, the decomposition of albumin is inhibited by the recycling system mediated by the Fc receptor, and this extends the half-life in the serum (Jan Terje Andersen and Inger Sandlie, Drug Metab. Pharmacokinet. 24 (4), 318 (2009)). Hence, pharmaceutical agents conjugated with albumin binding molecules are delivered efficiently to the tumor cells, and they can be effective over a long time. Such concepts are applied to pharmaceutical researches, and leading to the use of biomolecules binding to many natural or artificial albumins, such as the protein domain of bacteria or antibodies (S. C. Makrides, P. A. Nygren, B. Andrews et al., J. Pharmacol. Exp. Ther. 277 (1), 534 (1996); Lucy J. Holt, Amrik Basran, Kate Jones et al., Protein Eng. Des. Sel. 21 (5), 283 (2008)). Such molecules that bind to albumin are effective in extending the half-life in blood of pharmaceutical agents.

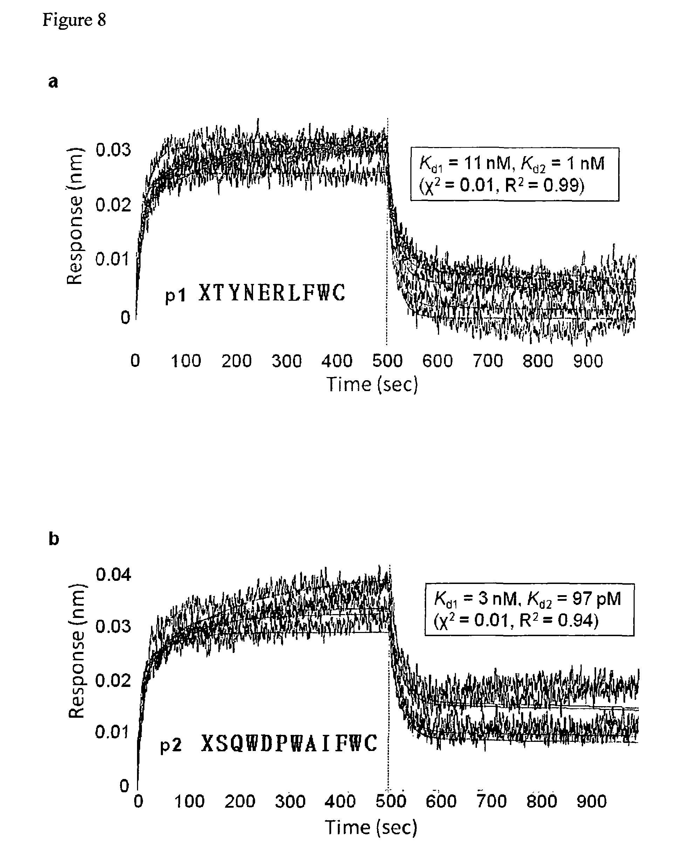

In the present invention, two cyclic peptides that bind to human serum albumin (HSA) were obtained using the Flexible Display Method in the Examples described hereinafter (Example 5, Fig. 5d, Fig. 8).
p1: XTYNERLFWC
p2: XSQWDPWAIFWC
(X is ClAB-L-Phe)

These cyclic peptides also fall within the scope of the present invention.

### 5. Explanation of General Technology and Terms used in the Present Invention

Please refer to the following on details of the molecular biological methods concerning the above explanations and the content of the Examples described hereinafter: Sambrook, Molecular Cloning: A Laboratory Manual, 3rd edition, Cold Spring Harbor Laboratory Press, 2001; Golemis, Protein-Protein Interactions: A Molecular Cloning Manual, 2nd edition, Cold Spring Laboratory Press, 2005.

In addition to the above, materials and methods for performing the present invention follow the conventional technology in the technical field of chemistry or molecular biology, and methods described in various general text books or specialized references are used. Many articles and patent documents published by the group of the present inventors are included in the specialized references. Such general text books or specialized references are incorporated herein.

Unless otherwise defined in the present specification, the scientific terms and technical terms used in relation to the present invention hold the same meaning as generally understood by a person skilled in the art. Further, the following explanation can be applied to terms used to describe the embodiments of the present invention. These definitions take the place of definitions in the text books or specialized reference incorporated herein when the text books or specialized references include an incoherent definition.

### Peptide, Proteins, Peptide Analogs, Non-Standard Peptides

Peptide is a collective name of biopolymer compounds formed by linking various amino acids through amide bond (peptide bond). Generally speaking, those relatively short chains having 50 amino acid residues or less in the chain are called peptides, and those with longer chains are called protein or polypeptide. Those compounds with 30 residues or less may also be called short chain peptides. In the present invention, the terms, short chain peptide, peptide, protein and polypeptide are used without any particular distinction, and they can take each other's place.

A standard (natural) peptide consists of 20 types of proteinous amino acids. In contrast, peptides that contain a partial structure that does not exist in the standard peptide are called nonstandard peptide or unnatural peptide or peptidomimetic or peptide analog, etc. Known examples of non-standard peptides existing naturally include vancomycin which is an antibiotic, or Cyclosporin A used as an immunosuppressive agent. In the present specification, arbitrary molecules including amino acids other than the 20 types of proteinous amino acid or hydroxy acids that are translated/synthesized by ribosome are called non-standard peptides or unnatural peptides or peptide analogs.

### Proteinous Amino Acid

A proteinous amino acid or natural amino acid is the 20 types of amino acids which are α-aminocarboxylate (or substituted α-aminocarboxylate) that are used in standard translation, alanine (Ala), valine (Val), leucine (Leu), isoleucine (Ile), proline (Pro), tryptophan (Trp), phenylalanine (Phe), methionine (Met), glycine (Gly), serine (Ser), threonine (Thr), tyrosine (Tyr), cysteine (Cys), glutamine (Gln), asparagine (Asn), lysine (Lys), arginine (Arg), histidine (His), aspartic acid (Asp), and glutamic acid (Glu).

### Non-Standard Amino Acid, Non-Standard Peptide

Amino acids in the present specification include both a proteinous amino acid and a non-standard amino acid, and peptides include non-standard peptides (or unnatural peptides or peptide analogs).

A "non-standard amino acid" refers to amino acid in general that differ in structure from the 20 types of proteinous amino acids used in natural translation, and it includes some hydroxy acids. In other words, it includes all of a non-proteinous amino acid or artificial amino acid, D-amino acid, N-methyl amino acid, N-acyl amino acid, β(beta)-amino acid, γ(gamma)-amino acid, δ(delta)-amino acid, which are proteinous amino acids whose side chain structure is chemically changed/modified in one part, or derivatives having a structure in which the amino group or carboxyl group on the amino acid back bone is substituted. Peptides in which non-standard amino acids are introduced, or the above "non-standard peptides", include polymers having these various non-standard amino acids as components. Non-standard peptides may be constituted partially or entirely of non-standard amino acids. Hence, non-standard peptides may have a main chain whose structure differs from standard amide bonds. For example, a depsipeptide consisting of an amino acid and a hydroxy acid, a polyester, or a N-methyl peptide in which continuous hydroxy acids are condensed, peptides having various acyl groups (acetyl group, pyroglutamic acid, fatty acid, etc.) on the N-terminus may be included as non-standard peptides. Further, non-standard peptides include cyclic peptides. A method for cyclization of the translated/synthesized straight chain peptide by intermolecular reaction is known (Goto et al., ACS Chem. Biol., 2008, 3, 120-129, WO 2008/117833 "PROCESS FOR SYNTHESIZING CYCLIC PEPTIDE COMPOUND"). One example is a cyclic peptide cyclized by thioether bond obtained by translation/synthesis of a peptide sequence in which a non-standard amino acid having a chloroacetyl group is placed on the N-terminus and a peptide sequence in which cysteine is placed in the peptide chain or the C-terminus. Various other structures can be used for cyclization according to various combinations of functional groups that can be bonded.

### Translation

Translation is generally to read the information of mRNA from the base sequence, and to convert it to an amino acid sequence on the ribosome, and it is substantially the same as protein (peptide) biosynthesis. A translation reaction is a reaction to synthesize peptide using a nucleic acid as the template, and various peptides can be synthesized by changing the sequence of the template cDNA or mRNA. A translation product is a peptide (including peptide, protein, peptide analog, non-standard peptide) synthesized by ribosome.

Cell free translation is a technology to perform artificial translation/synthesis in a test tube, and it is also called in vitro protein synthesis. The cell-free translation system is a concept that includes both the method and product (a solution-like mixture or a kit) for translation/synthesis, but when the term is used in the Claims, it should be taken to mean an invention of a "product" used in translation. The transcription-linker association-translation coupling reaction system and the linker association-translation coupling system are also written as product inventions.

### Annealing, Hybridization

Annealing is generally a process of a degenerated single stranded DNA becoming a duplex molecule through formation of a complementary base pair under a suitable condition. In the present specification, annealing refers to complementary base pairs of a single strand of a structure having a nucleic acid base as the side chain, not just DNA, associating with each other to form a duplex. Formation of a complementary base pair is referred to as hybridization or complementary base pairing, and it is a pairing of nucleic acid bases in a complementary combination by a hydrogen bond. Hybridization is a hybrid (a hybrid duplex nucleic acid molecule) formation from a single stranded DNA or RNA through formation of a complementary base pair. In the present specification, the linker base sequence and the complementary sequence section of mRNA form a duplex through annealing. The complementary base pairing is a natural bond of polynucleotides (RNA or DNA, or other straight chain molecules having nucleic acid base as the side chain) under an acceptable salt and temperature condition, and the complementation of the two single stranded molecules may be "partial," in which case only some of the bases in the nucleic acid sequence form a bond, and the ratio of the complementary base pair can be shown by numeric values. When a complete complementation exists between two types of single stranded molecules, the complementation is "complete" (100%). The degree of complementation significantly affects the efficiency and strength of hybridization. Various methods well known to a person skilled in the art can be used to determine whether two types of nucleic acid molecules would hybridize. Typically, the combination of the bases of nucleic acids that form a complementary base pair are adenine (A) and thymine (T) or uracil (U), and guanine (G) and cytosine (C). Thymine (T) and uracil (U) are used interchangeably according to the type of polynucleotide (DNA or RNA). Further, a so-called non-Watson-Crick base pair such as G-U may also exist as a thermodynamically stable base pair, so such pair may also be called complementary.

### Library

Libraries are groups of multiple molecules (e.g. multiple nucleic acids, translation products, translation product/linker/mRNA complexes, translation product/linker/mRNA-cDNA complex molecules). Since the library in the present invention is created for the sake of preparing a wide range of possible genes to select those with the targeted phenotypes therefrom with an aim to create peptides, proteins, and peptide analogs with desired functions and characteristics, so it is preferable to use a library with a large number of candidate molecules. The variety of the library may be expressed as 10¹¹ units of different gene sequences, typically 10¹²-10¹³ units, preferably 10¹⁵ units of different gene sequences.

### Selection

Selection is to substantially separate a certain molecule from other molecules in the library. In evolution molecule engineering, a peptide library is obtained as a translation product from the gene library (a group of molecules in which peptides and nucleic acids are associated), and molecules with desired functions or characteristics are selected by the screening system from the peptide library. Each selected peptide molecule has a nucleic acid which is its gene attached to it like a tag. A nucleic acid library is prepared by PCR amplification of the nucleic acid section of the selected associated molecule to obtain a biased library including many clones with the targeted phenotype, then a similar selection experiment is performed again. By repeating the selection-amplification operation, molecules with the desired phenotype becomes condensed (enriched) in the library.

The present invention is described in detail by Examples below. These Examples are presented to explain the present invention, and they do not limit the scope of the present invention.

### EXAMPLES

The present Examples compared the stability of the translation product-Pu-linker/mRNA complex formed by ligation used in the conventional mRNA display method and the translation product-Pu-linker/mRNA complex formed by annealing. In addition, the present Examples compared the formation efficiency of the library in which translation products are random peptides. Then, the present Examples confirmed that the T7-tag peptide (T7-peptide), which is the model translation product, can be selectively condensed from a random DNA pool. Further, the Examples actually performed a selection experiment of functional peptides and proved the usefulness of selection using the TRAP.

### Example 1: Stability of peptide-Pu-linker/mRNA complex

The bond between the linker and mRNA in the method of the present Examples is a non-covalent bond. A T7-peptide pull-down assay was performed to confirm that neither the bond between the peptide and mRNA via the linker is dissociated nor is the mRNA replaced with other unrelated mRNAs in between the formation of a peptide-linker/mRNA complex and the peptide aptamer selection.

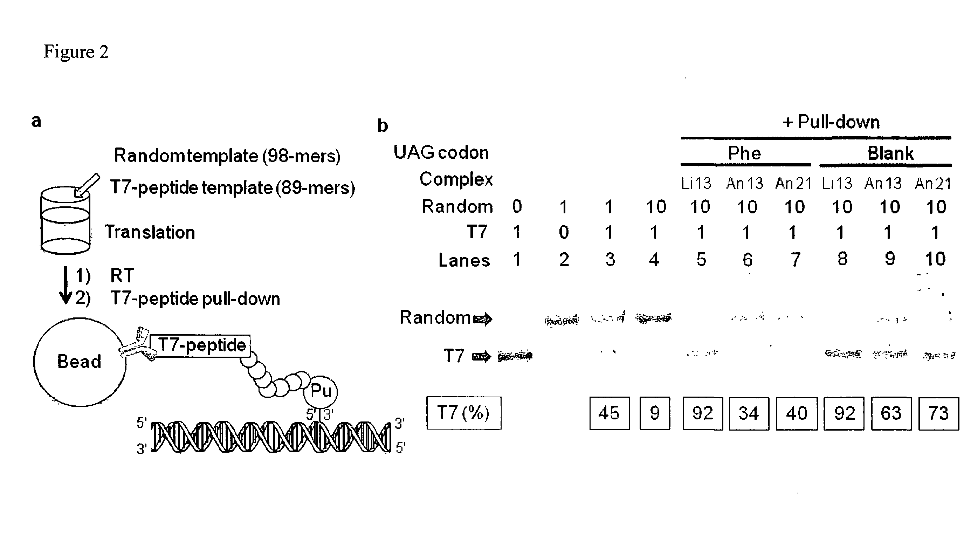

The scheme of this assay is shown in Fig. 2a. The Pu-linker/mRNA complex of mRNA encoding T7-peptide and mRNA encoding a random sequence peptide (these mRNAs have the same Pu-linker annealing sequence at 3'-UTR) are mixed in a ratio of 1:10, and after translation and reverse transcription (RT) using a template mRNA mixture, the T7-peptide-Pu-linker/mRNA/cDNA complex was selectively recovered using anti-T7 antibody-immobilized beads.

The analysis result obtained by using electrophoresis after amplifying cDNA of the selected complex is shown in Fig. 2b. Lanes 1-4 include a marker, random mRNA, and mixtures of T7-mRNA and random mRNA at a ratio of 1:1 or 1:10 synthesized from mRNA of DNA T7. In Lanes 5-10, mixtures of T7- mRNA and random mRNA at a ratio of 1:10 were added to the translation liquid. The reaction was performed in a translation solution in which the UAG codon was assigned Phe (Lanes 5-7) or was made blank (Lanes 8-10). Then, the mRNA templates of the following types were used in reaction: Lanes 5 and 8, mRNA of the Li13-type; Lanes 6 and 9, mRNA of the An13-type; Lanes 7 and 10, mRNA of the An21-type.

Firstly, the stability of the peptide-Pu-linker/mRNA complex formed by ligation used in the conventional mRNA display method (Fig. 1c, Li13) and the peptide-Pu-linker/mRNA complex (Fig. 1c, An13) formed by annealing was compared. The result is shown in Lanes 5-7 of Fig. 2b.

This experiment is performed by using Phe-tRNA_{CUA} aminoacylated by flexizyme to designate Phe to the UAG codon. Mostly T7-cDNA was recovered in the selection of peptides using the peptide-Pu-linker/mRNA complex (Li13) through ligation, but a large amount of random-cDNA was present in the peptide-Pu-linker/mRNA complex (An13) formed by annealing of 13mer. The experiment result shows that the complex of Li13 is stable, but the complex of an13 is unstable. Hence, an An21-type Pu-linker/mRNA in which the number of base pairs was increased from 13 to 21-mer was prepared to stabilize the formation of a duplex between the Pu-linker DNA and mRNA (Fig. 1b). However, even this improvement merely changed the percentage of T7-cDNA in the recovered cDNA "T7-cDNA/(T7-cDNA+random cDNA)" from 34% to 40% (Fig. 2b, comparison of Lanes 6 and 7). This result shows that more than half of the peptide-Pu-linkers were dissociated from mRNA during one of the processes of translation reaction, RT or T7 peptide pull-down. Although data will not be presented herein, we assumed that dissociation occurred during the translation reaction, since the Pu-linker DNA/mRNA duplex was stable during the RT and T7 peptide pull-down processes. Hence, the present inventors performed the following experiment based on the assumption that the helicase activity of ribosome which induces the translation reaction causes the dissociation.

A UAG codon was placed immediately before the Pu-linker DNA/mRNA duplex section on the mRNA with the intention of halting ribosome before the section to reduce the effect of helicatase activity, and Phe-tRNA^{Asn-E2} _{CUA} was removed from the translation mixture to make UAG a blank codon (Fig. 1 b).

As a result, the percentage of T7-cDNA in the recovered cDNA "T7-cDNA/(T7-cDNA+random cDNA)" changed from 34% to 63% in the complex of the An13-type, and from 40% to 73% in the An21-type complex (Fig. 2b, comparison of Lanes 6 and 9, and Lanes 7 and 10). This result suggests that the main reason of the dissociation of the T7-peptide-Pu-linker from T7-mRNA is the helicase activity of ribosome. It was shown through experiment that T7-peptide-Pu-linker/mRNA complex is stabilized by extending the length of the Pu-linker DNA/mRNA duplex and inserting a blank codon immediately before the duplex section.

### Example 2: Efficiency in forming a random sequence peptide-Pu-linker/mRNA complex

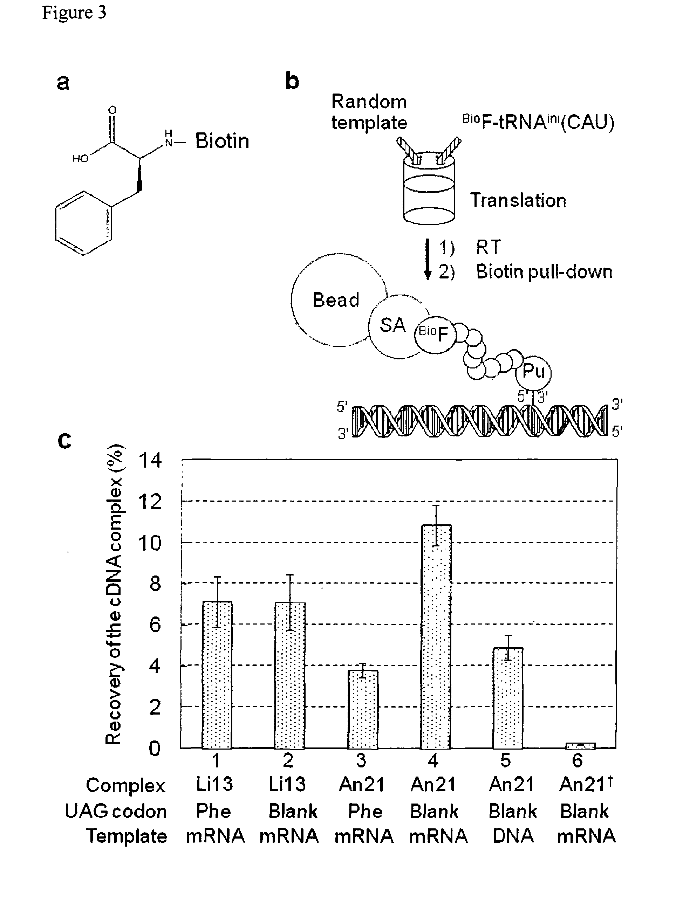

In the selection of peptides from the random library, the variety of the library is determined by the efficiency of formation of the complex that links peptide and mRNA. To assess the efficiency of complex formation, an initiation tRNA^{fMet}_{CAU} to which biotinized Phe (Fig. 3a) binds to was added to the translation system excluding Met to synthesize a peptide labeled with biotin on the N-terminus (Fig. 3b). Accordingly, it is possible to selectively recover just mRNAs that display biotinized peptides by using streptavidin-immobilized beads (SA-beads).

The result of quantifying the recovered complexes is shown in Fig. 3c. The recovery of cDNA complexes was calculated by dividing the amount of recovered cDNA by the theoretical value of the mRNA/Pu-linker (1 µM) amount in the reaction solution. The error bar shows the standard error calculated from the three experiments. The reaction was performed in a translation liquid in which the UAG codon was assigned Phe (1^{st}, 3^{rd} lane) or made blank (2^{nd}, 4^{th}-6^{th} lanes). The following types of templates were used for the reaction: Lanes 1-2, mRNA of the Li13-type; Lanes 3-4, mRNA of the An21-type; Lane 5, DNA of the An21-type; Lane 6, DNA template of the An21-type at 37°C without incubation.

The efficiency of forming a random sequence peptide-Pu-linker/mRNA complex by the Pu-linker of the conventional mRNA Display method and the Pu-linker to capture mRNA by annealing was compared by adding the Pu-linker/mRNA complexes of the Li 13 type or An21 type as a template into the translation system. The cDNA recovered by a biotin pull-down assay was quantified by real-time PCR. The recovery ratio for the reaction system in which the UAG codon was assigned Phe or the reaction system of a blank codon did not change at 7% in the Li13 type mRNA (Fig. 3c, Lane 1 and Lane 2). However, in the An21 type mRNA, the recovery ratio was 4% for the reaction system in which the UAG codon was assigned Phe and it was 11% in the reaction system of a blank codon (Fig. 3c, Lane 3 and Lane 4). This shows that in a reaction system in which the UAG codon was assigned Phe, the Pu-linker DNA/mRNA duplex in the An21 type Pu-linker/mRNA complex was dissociated by the ribosome before puromycin started attacking the nascent peptide. It is also considered that placing a blank codon immediately before the duplex section stalls the ribosome at the blank codon, and suppresses dissociation by ribosome of the Pu-linker DNA/mRNA duplex in the Pu-linker/mRNA complex. It is considered that the poor recovery ratio of the Li13 type mRNA compared to the An21 type mRNA (Fig. 3c, Lane 2 and Lane 4) results from the fact that the amount of Li13-type Pu-linker/mRNA that is properly ligated is small.

Next, the efficiency of forming the An21 type Pu-linker/mRNA complex was assessed using a template of DNA instead of mRNA.

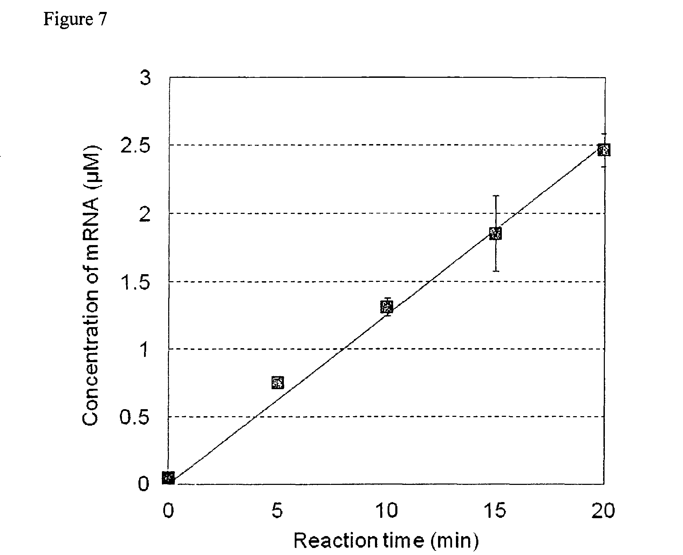

To speed up the selection process, it is desirable that amplified template DNA does not require purification in each round. Hence, assessment was made to optimize the composition of the translation reaction solution of the transcription couple type. The PCR mixture containing DNA that has been subjected to reverse transcription and amplification was added to the transcription/translation solution in its original form without purification, and the obtained mRNA was quantified by RT real-time PCR. The transcription/translation solution was prepared by mixing solA and solC (refer to Tables 2 and 3 mentioned hereinafter). A crude solution 5% (v/v) containing random-DNA after RT-PCR reaction was added to the liquid, and a transcription/translation reaction (1 µL) was performed at 37°C for 20 min. The amount of mRNA at each point in time is shown in Fig. 7. The generation of a sufficient amount of mRNA (<1 pmol/µL) was confirmed in the reaction solution containing 1 µM of T7 RNA polymerase after 5-10 min. of incubation.

An21 type random DNAs were added as templates to the reaction solution to perform a biotin pull-down assay. As a result, the recovery ratio became about a half compared to that of mRNA, but it is about the same level as the conventional mRNA display method (Fig. 3c, Lanes 2, 4, 5). This shows that the TRAP system, which is a transcription-linker association-translation coupling reaction system, has a display efficiency that can withstand commercial use. Based on these results, it was determined that an An21-type random mRNA should be used in the selection in the first round to secure library variety, and amplified DNA should be used as a template in the subsequent rounds to speed up selection. Such selection using a linker captured by annealing, and using the transcription-translation coupling system (TRAP system) using an amplified DNA as the template in the second round and thereafter was named the Flexible Display Method.

### Example 3: Selective Condensation of T7-peptide-Pu-linker/mRNA complex

The condensation efficiency of T7-DNA was compared by performing the above T7 peptide pull-down assay in the system using the An21 type linkers and the conventional mRNA Display method (Li 13 type linkers).

The Pu-linker/mRNA complexes of T7-mRNA and random mRNA were mixed at a ratio of 1:3000, and they were added to the translation solution. The RT-PCR product was analyzed after pull-down assay.

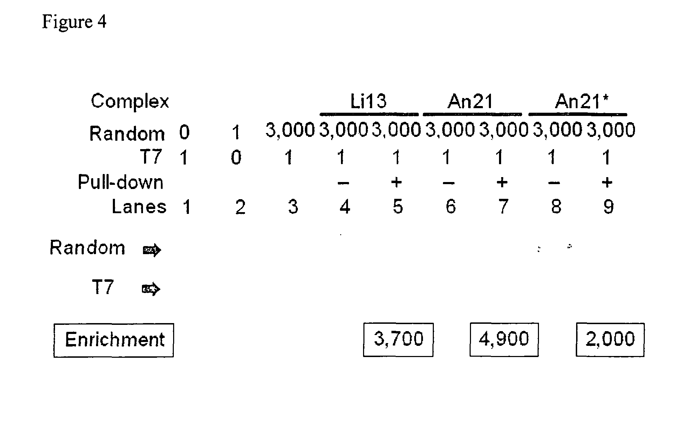

The result is shown in Fig. 4. From the band strength on the gel of the sample after pull-down, the condensation efficiency of the T7-peptide template was calculated. Lanes 1-3 include DNA markers synthesized from T7-mRNA, random mRNA, and a mixture of T7-mRNA and random mRNA at a ratio of 1:3000. T7-mRNA and random mRNA (or DNA) were mixed at a ratio of 1:3000 in a translation system in which the UAG codon is assigned Phe (Lanes 4 and 5) or made blank (Lanes 6-9). The following types of templates were used in the reaction: Lanes 4-5, mRNA of Li13-type; Lanes 6-7, mRNA of An-21 type; Lanes 8-9, DNA of An21-type.

The T7-mRNA was condensed to 3700 folds the original (Fig. 4, Lanes 4 and 5) in the conventional mRNA display method, and it was condensed to 4900 folds the original (Fig. 4, Lanes 6 and 7) in the system using An21-type linkers. From the result, it was clearly seen that a translation system incorporating linkers captured by annealing using a mRNA template can selectively condense DNAs of specific peptides (T7-DNA) at an efficiency that compares with or exceeds the conventional mRNA Display method.

Experiments were performed in which DNA is used as a template for selective condensation of T7peptide-Pu-linker/mRNA complex. The T7-DNA and random-DNA were added to the transcription/translation reaction system (TRAP system) at a ratio of 1:3000. The result of the T7 peptide pull-down assay showed that the T7-DNA was condensed to 2000 folds the original (Fig. 4, Lanes 8 and 9). Hence, it was shown that the polypeptide-Pu-linker/mRNA can be generated not just from the mRNA template but also from the DNA template in a system using linkers that capture mRNA by annealing, and it was also shown through a pull-down experiment using target-immobilized beads that specific polypeptides can be condensed.

### Example 4: In vitro Selection of HSA-bonded Peptides

The Flexible Display Method was used to select peptide aptamer binding to human serum albumin (HSA) from a library of cyclic peptides.

N-[3-(2-chloroacetamide)benzoyl]-L-phenylalanine (abbreviation: ClAB-L-Phe), which is a new amino acid for cyclization, was synthesized to create a cyclic peptide library (Fig. 5a), and an AUG initiation codon was assigned to this amino acid.

An mRNA library was created concerning peptide consisting of 8 to 12 random amino acid sequences having ClAB-L-Phe and Cys at both ends. The encoded peptide is voluntarily cyclized by intermolecular thioether bond formed between a chloroacetyl of ClAB-L-Phe and the thiol group of Cys (Fig. 5b).

A library was created of cyclic peptide displayed on the An21 type Pu-linker/mRNA complex in the translation system, and it was used for the pull-down assay against HSA immobilized to the SA beads. DNA products that had been amplified by RT-PCR and recovered were directly added to the TRAP system without purification, and the obtained cyclic peptide library was used in the subsequent round of selection. The recovery of cDNA increased significantly in the fourth round of selection (Fig. 5c), so washing was conducted under a more stringent condition in the subsequent two rounds to condense bound peptides of a high affinity. It should be noted that the six rounds required for selection was performed in about 14 hours, actually proving that the display method using the TRAP system (Flexible display method) speeded up the peptide selection.

Furthermore, selection of a similar cyclic peptide bonding to HSA was performed using the conventional mRNA display method (Fig. 5c), and the sequences selected by the mRNA display method and the Flexible display method were compared, providing a result that the two most highly obtained sequences were the same (Fig. 5d, p1: XTYNERLFWC and p2: XSQWDPWAIFWC, wherein X is ClAB-L-Phe). Accordingly, the Flexible display method was proven to have a performance equivalent to that of the mRNA display method.

Derivative peptides having reverse and shuffled sequences of the selected peptides (p1 and p2) and the original peptide were prepared as the An21-type complexes. The obtained complexes were subjected to pull-down using the HSA-immobilized SA beads and the SA beads free of HSA, and the recovered cDNA was quantified, which provided the result that p1 and p2 actually bond with HSA (Fig. 5e). Furthermore, since the bonding activity to HSA is lost when the amino acid sequence of the obtained cyclic peptide is reconfigured (reversed or shuffled), it could be seen that the bond of peptide to HSA is sequence-dependent (Fig. 5e).

Next, these peptides were chemically synthesized by Fmoc solid phase synthesis, and the bond to HSA was assessed using the Bio-Layer Interferometry method (BLI method). A bonding assay was performed under the condition of 50 mM Hepes-KOH pH 7.5, 300 mM NaCl, 0.05% Tween20 and 0.001 % DMSO at 30°C. Each step of the assay consists of the bond reaction of peptide to HSA being 500 s, and the dissociation reaction being 500 s. The peptide solution was created by the following dilution series (p1: 16, 12, 8, 2, 1 nM, and p2: 16, 12, 8, 4 nM).

This experiment also showed that p1 and p2 bond to HSA (Fig. 8).

### Example 5: Confirmation of the generation amount of associated molecule under the presence/absence of EF-P

An mRNA was prepared from DNA of the following sequence using a common method.

### (SEQ ID No.34-(NNW)₈-SEQ ID No.35)

N is A/T/G/C, and W is A/T. When mRNA is translated using Flexiyme to introduce N-α-biotinyl-L-phenylalanine to the initiation ATG codon, and tryptophan to the elongation ATG codon, the resulting amino acid sequence in which NNW is not the termination codon, TAA or TGA, is expected to be as follows.
Biotin-Phe-Xaa-Xaa-Xaa-Xaa-Xaa-Xaa-Xaa-Xaa-Trp-Gly-Gly-Gly-Ser-Gly-Ser

### (Biotin-Phe-(Xaa)₈-SEQ ID No.36)

In this sequence, Xaa corresponds to the 18 types of natural amino acids other than methionine, tryptophan.

The mRNA is mixed with 1.1 equivalent amount of Puromycin-linker.an21, and the mixture is added to the translation liquid to achieve a final concentration of 2 µM, then it was reacted at 37°C for 25 min. The translation liquid includes 19 types of natural amino acids other than methionine and ARS corresponding thereto, N-α-biotinyl-L-phenylalanine-tRNA (initiation CAU) and L-tryptophan-tRNA (elongation CAU) prepared with flexiyme, and it was translated under two conditions, in one system incorporating 3 µM of EF-P and in another system without any EF-P. EDTA was added and reverse transcription was conducted by a common method, then a part of the result was mixed with streptavidin beads suspension to be agitated for 30 min. The mixture was washed 3 times after the supernatant was removed, a tris hydrochloride buffer containing 0.05% Tween20 was added and heated at 95°C for 5 min. after which the supernatant was removed, then cDNA was quantified by real-time PCR. The recovery ratio of cDNA calculated as the generation ratio of the associated molecule was 14% in a system free of EF-P, and 10% in a system incorporating EF-P. The result showed that EF-P can be added without hindering the normal reaction system.

### [Material and Method]

### Abbreviations

CME, cyanomethyl ester; MgSO₄, magnesium sulfate; MgCl₂, magnesium chloride; DMSO, dimethyl sulfoxide; Tris, Tris(hydroxymethyl)aminomethane; Hepes, 2-ethansulfonic acid; EDTA, ethylene diaminetetraacetic acid; DTT, dithiothreitol; KOH, potassium hydroxide

### Preparation of Aminoacyl-tRNA

The L-Phe-tRNA^{Asn-E2}_{CUA} and biotin-L-Phe-tRNA^{fMet}_{CAU} were prepared by the method mentioned above. N-[3-(2-chloroacetamide)benzoyl]-L-Phe-CME (ClAB-L-Phe-CME) was prepared by coupling L-Phe with 3-(2-chloroacetamide)benzoyl chloride and performing cyanomethyl esterification by a method similar to that mentioned above for synthesis. The ClAB-L-Phe-tRNA^{fMet}_{CAU} was prepared by a method similar to that mentioned above. (H. Murakami, A. Ohta, H. Ashigai et al., Nat. Methods 3 (5), 357 (2006); Y. Goto, T. Katoh, and H. Suga, Nat. Protoc. 6 (6), 779 (2011).)

### Preparation of T7-DNA, random-DNA, T7-mRNA, and random mRNA

The template DNA was synthesized by an elongation reaction and PCR using oligonucleotide shown in Table 1.

[Table 1]

**Table 1. Sequence of oligonucleotides. The oligonucleotides were purchased from Greiner Bio-One or Operon or BEX. SPC18: 18-O-Dimethoxytritylhexaethyleneglycol, 1-[(2-cyanoethyl)- (N,N-diisopropyl)]-phosphoramidite.**

| Name | Sequences | SEQ ID NO |
|---|---|---|
| T7SD8M2.F44 | | No.1 |
| G5S-4an21.R41 | | No. 2 |
| G5S-4an13.R36 | | No. 3 |
| G5S-4.R20 | 5'-TAGCT ACCTC CTCCT CCACC-3' | No. 4 |
| Biotin-DNA | 5'-CCCGC CTCCC GCCCC CCGTC C-biotin-3' | No. 5 |
| Competitor DNA | 5'-CCCGC CTCCC GCCCC CCGTC C-3' | No. 6 |
| Puromycin-linker.an13 | | No. 7 |
| Puromycin-linker.an21 | | No. 8 |
| SD8NNK8CG5S4.R69 | | No.9-(MNN)₈-No.10 |
| SD8NNK9CG5S4.R72 | | No.9-(MNN)₉-No.10 |
| SD8NNK10CG5S4.R75 | | No.9-(MNN)₁₀-No.10 |
| SD8NNK11CG5S4.R78 | | No.9-(MNN)₁₁-No.10 |
| SD8NNK12CG5S4.R81 | | No.9-(MNN)₁₂-No.10 |
| SD8T7G5S4.R72 | | No. 11 |
| SD8No38revG5S4.R69 | | No. 12 |
| SD8No38ranG5S4.R69 | | No. 13 |
| SD8No41revG5S4.R75 | | No. 14 |
| SD8No41ranG5S4.R75 | | No. 15 |

N is a base selected from A, T, G, C; M is a base which is either A, or C.

The following oligonucleotides were used for elongation.
Random-DNA: T7SD8M2.F44, and one of SD8NNK8CG5S4.R69-SD8NNK12CG5S4.R81 T7-DNA: T7SD8M2.F44, and SD8T7G5S4.R72

The following oligonucleotides were used as the primer for PCR.
T7SD8M2.F44 and G5S-4an13.R36 (Li13, An13) or G5S-4an21.R41 (An21)

mRNA was synthesized from template DNA using T7 RNA polymerase. When selecting a peptide bonding to HSA, mRNA that has been purified by isopropanol precipitation was used, and mRNA purified by electrophoresis was used for other experiments.

### Conventional Cell-free Translation System and the TRAP System

The creatine kinase, creatine phosphate and Escherichia coli tRNAs were purchased from Roche Diagnostics (Tokyo, Japan). Myokinase was purchased from Sigma-Aldrich Japan (Tokyo, Japan). Chemical substances, proteins and ribosome used for translation were prepared by the same method as that used in previous articles (K. Josephson, M. C. T. Hartman, and J. W. Szostak, J. Am. Chem. Soc. 127 (33), 11727 (2005); Y. Shimizu, A. Inoue, Y. Tomari et al., Nat. Biotechnol. 19 (8), 751 (2001); Hiroyuki Ohashi, Yoshihiro Shimizu, Bei-Wen Ying et al., Biochem. Biophys. Res. Commun. 352 (1), 270 (2007). Patrick C. Reid, Yuki Goto, Takayuki Katoh et al., Methods Mol. Biol. 805, 335 (2012).)

The concentration of the proteinous factor and ribosome in the stock solution B (solB) and the final concentration in the reaction solution are shown in Table 2.

[Table 2]

**Table 2. Concentration of proteinous factors and libosomes included in solB. The concentration of the reaction solution is shown in the right column. solC was created by mixing 100 µl of solB and 10 µL of 90 µM T7 RNA polymerase.**

| Name | HsolB stock solution (µM) | Reaction mixture (µM) |
|---|---|---|
| AlaRS | 6.1 | 0.61 |
| ArgRS | 0.25 | 0.025 |
| AsnRS | 3.2 | 0.32 |
| AspRS | 1.1 | 0.11 |
| CysRS | 0.17 | 0.017 |
| GlnRS | 0.50 | 0.05 |
| GluRS | 1.9 | 0.19 |
| GlyRS | 0.75 | 0.075 |
| HisRS | 0.17 | 0.017 |
| IleRS | 3.3 | 0.33 |
| LeuRS | 0.33 | 0.033 |
| LysRS | 0.92 | 0.092 |
| MetRS | 0.25 | 0.025 |
| PheRS | 5.7 | 0.57 |
| ProRS | 1.3 | 0.13 |
| SerRS | 0.33 | 0.033 |
| ThrRS | 0.75 | 0.075 |
| TrpRS | 0.25 | 0.025 |
| TyrRS | 0.17 | 0.017 |
| ValRS | 0.17 | 0.017 |
| Methionyl-tRNA formyltransferases | 5.0 | 0.5 |
| Initiation factor 1 | 23 | 2.3 |
| Initiation factor 2 | 3.3 | 0.33 |
| Initiation factor 3 | 13 | 1.3 |
| Elongation factor G | 2.2 | 0.22 |
| Elongation factor Tu | 83 | 8.3 |
| Elongation factor Ts | 83 | 8.3 |
| Release factor 2 | 2.1 | 0.21 |
| Release factor 3 | 1.4 | 0.14 |
| Ribosome recycling factor | 4.2 | 0.42 |
| Nucleoside-diphosphate kinase | 0.83 | 0.083 |
| Inorganic pyrophosphatase | 0.83 | 0.083 |
| T7 RNA polymerase | 0.83 | 0.083 |
| Creatine kinase | 33 (µg/mL) | 3.3 (µg/mL) |
| Myokinase | 25 (µg/mL) | 2.5 (µg/mL) |
| Ribosome | 10.00 | 1 |

The concentration of tRNA and other small molecules in the stock solution A (solA) and the final concentration in the reaction solution are shown in Table 3.

[Table 3]

**Table 3. Concentration of the compound in solA and tRNA. Concentration of the reaction solution is shown in the right column.**

| Name | HsolA stock solution (mM) | Reaction mixture (mM) |
|---|---|---|
| ATP | 18.3 | 2.0 |
| GTP | 18.3 | 2.0 |
| CTP | 9.2 | 1.0 |
| UTP | 9.2 | 1.0 |
| Creatine phosphate | 183 | 20.1 |
| Hepes-KOH pH 7.6 | 459 | 50.5 |
| Potassium acetate | 917 | 100.9 |
| Magnesium acetate | 110 | 12.1 |
| Spermidine | 18.3 | 2.0 |
| DTT | 9.2 | 1.0 |
| 10-formyl-5,6,7,8 tetrahydrofolic acid | 0.9 | 0.1 |
| *E. coli* tRNA mix | 14 (mg/mL) | 1.54 (mg/mL) |

100 µL of solB and 10 µL of 90 µM T7RNA polymerase were mixed to create solC.

The reaction solution of the conventional cell-free translation system was prepared by mixing solA (11%, v/v) and solB (10%, v/v) with other solutions. The reaction solution of TRAP system was prepared by mixing solA (11%, v/v), solC (11%, v/v), and Pu-linker (final concentration 1.1 µM) with other solutions.

### Preparation of a Li13- An13- An21-type Pu-linker/mRNA complex

The Li 13-type Pu-linker/mRNA complex was prepared by mixing Puromycin-linker.an13 of Table 1 with mRNA to form a covalent bond by T4 RNA ligase. Pu-linker/mRNA complexes of the An13-type and the An21-type were prepared by mixing mRNA and a 1.1 equivalent amount of Puromycin-linker.an13 or Puromycin-linker.an21 of Table 1. The concentration of Pu-linker/mRNA complex shown below is the mRNA concentration.

### Analysis of Stability of T7-peptide-Pu-linker/mRNA/cDNA complex

A translation solution containing 20 types of proteinous amino acid each at 0.25 mM, Li13-, An13- or An21-type Pu-linker/T7-mRNA complexes, and corresponding Pu-linker/random mRNA complexes at 1 µM was used. In a different method, 25 µM 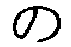 Phe-tRNA^{Asn-E2}CUA was added to the reaction solution to reassign the UAG code to Phe from blank.

The translation and reverse transcription reactions were performed as follows. The translation solution (2 µL) was incubated at 37°C for 15 min. After 1 µL of 50 mM EDTA (pH 7.5) was added, the reaction solution (3 µL) was added to 1 µL of 4×RT mix: 200 mM Tris-HCl (pH 8.3), 300 mM KCl, 75 mM MgCl₂, 4 mM DTT, 2 mM each of dNTPs (dATP, dTTP, dGTP, dCTP); 10 µM G5S-4.R20, 6 U ReverTraAce (TOYOBO). The final concentration of the mRNA template and the primer are as follows: 0.05 µM T7-mRNA, 0.5 µM random-mRNA, 2.5 µM G5S-4.R20. The reaction solution was incubated at 42°C for 30 min.

The T7-peptide pull-down was performed as follows. The reverse transcription reaction solution was diluted to 10 folds with HBST (50 mM Hepes-KOH pH7.5, 300 mM NaCl, 0.05% Tween20), and the diluted reaction solution (10 µL) was mixed with the anti-T7 peptide antibody (MBL) immobilized on the Dynabeads Protein G (VERITAS), and incubated at 4°C for 15 min. The beads were washed 3 times with 10 µL of HBST, and suspended in 25 µL of 0.5×PCR buffer [5 mM Tris-HCl pH 8.4, 25 mM KCl, 0.05%(v/v) Triton X-100]. The solution was heated at 95°C for 5 min, and 1 µL of supernatant was added to 19 µL of 1×PCR mixture (1×PCR buffer, 2 mM MgCl₂, 0.25 m each of dNTPs, 0.25 µM T7SDM2.F44, 0.25 µM G5S-4.R20 and Taq DNA polymerase). The DNA amplified by PCR (20 s at 94°C, 20 s at 60°C, 30 s at 72°C) was analyzed by electrophoresis.

### Quantification of the Creation of Random Peptide-Pu-Linker/mRNA Complex

A translation reaction solution containing nineteen types of proteinous amino acids (excluding methionine) at 0.25 mM each, 25 µM of biotin-Phe-tRNA^{fMet}_{CAU} and 1 µM of Pu-linker/random mRNA complexes of either the Li13-type or the An21-type was used. In a different method, 25 µM of Phe-tRNA^{Asn-E2}_{CUA} was added to the reaction solution, and the UAG codon that was blank was reassigned Phe. A reaction solution for the TRAP system containing nineteen types of proteinous amino acids (excluding methionine) at 0.25 mM each, 25 µM of biotin-Phe-tRNA^{fMet}_{CAU} and 5% (v/v) random-DNA RT-PCR solution was also used.

Translation (2 µL) and reverse transcription (4 µL) was performed by the above method. The biotin-random peptide-Pu-linker /mRNA/cDNA complex was recovered using a streptavidin magnetic beads (SA-beads), and the recovered cDNA was quantified by real-time PCR. The reaction solution containing reverse-transcribed random cDNAs was continuously diluted and used as the standard.

### Selective Condensation of T7-cDNA using Embodiments of Conventional mRNA Display Method and Flexible Display Method

A translation solution containing 20 types of proteinous amino acids at 0.25 mM each, 0.3 nM of Pu-linker/ T7-mRNA complexes of either the Li13-type or the An21-type and corresponding Pu-linker /mRNA/cDNA complexes at 1 µM was used. 25 µM of Phe-tRNA^{Asn-E2}_{CUA} was added for the conventional mRNA display method (Li13), and the UAG codon that was blank was reassigned Phe. A reaction solution of the TRAP system containing 20 types of proteinous amino acids at 0.25 mM each, a 5% (v/v) RT-PCR solution that mixes T7-cDNA and random cDNA at a ratio of 1:3000 was also used.

Translation (2 µL), reverse transcription (4 µL), T7-peptide pull-down assay and analysis by electrophoresis were performed as indicated above.

### In vitro Selection of HSA-Binding Peptides using TRAP Display

In the first round, a translation reaction solution (50 µL) containing 19 types of proteinous amino acid (-Met) at 0.25 mM each, 10 µM of ClAB-L-Phe-tRNA^{fMet}_{CAU}, 1 µM of mRNA library and 1.1 µM Pu-linker (an21) was incubated at 37°C for 15 minutes. After adding 12.5 µL of 100 mM EDTA (pH 7.5), the liquid reaction mixture was incubated at room temperature for 20 min. with the HSA immobilized SA-beads (1.6 pmol HSA). The beads were washed three times with 60 µL of HBST, and suspended in 5 µL of 1×RT mixture [1 µM G5S-4.R20, 50 mM Tris-HCl pH 8.3, 75 mM KCl, 3 mM MgCl₂, 10 mM DTT, 0.5 mM each of dNTPs, 5 U M-MLV (+)(Promega)]. Then the mixture was incubated at 42°C for 30 min., and subsequently added to 100 µL of 1×PCR mixture (1×PCR buffer, 2 mM MgCl₂, 0.25 mM each of dNTPs, 0.25 µM T7SDM2.F44, 0.25 µM G5S-4.R41, and Taq DNA polymerase) and heated at 95°C for 5 min. The 1 µL of mixture was used for quantification of the recovered cDNA, and the remainder was added to Taq DNA polymerase to be subjected to PCR (at 94°C for 20 s, at 55°C for 20 s, at 72°C for 30 s).

In the second and subsequent rounds, a liquid translation mixture (20 µL) of the TRAP system containing 19 types of proteinous amino acid (excluding Met) at 0.25 mM each, 25 µM of ClAB-L-Phe-tRNA^{fMet}_{CAU}, and 5% (v/v) of PCR crude solution was incubated at 37°C for 15 minutes. After adding 5 µL of 100 mM EDTA (pH 7.5), the liquid reaction mixture was added to 8.5 µL of 4×RT mixture [200 mM Tris-HCl (pH 8.3), 300 mM KCl, 75 mM MgCl₂, 4 mM DTT, 2 mM each of dNTPs, 10 µM G5S-4.R20, 6 U ReverTraAce] and incubated at 42°C for 30 min. 6 µL of 100 mM EDTA (pH 7.5) and 4 µL of 500 mM Hepes were added, and the reaction was terminated, then the solution was mixed with the HSA-immobilized SA beads (1.1 pmol HSA), and the mixture was incubated at 25°C for 10 min. The beads were washed three times with 60 µL of HBST, then the beads were recovered and mixed with 40 µL of 1×PCR mixture, and the solution was heated at 95°C for 5 min. The recovered cDNAs were quantified by real-time PCR, and the cDNA amplification by PCR was performed similarly to the first round.

The third and subsequent rounds reduced the reaction solution to a 1/4 scale. In addition, a negative selection using a streptavidin beads was performed three times before the positive selection. In the fifth and sixth rounds, a more stringent washing using 200 µL of HBST was performed as the second washing at 37°C for 30 min. After the sixth round, the amplified cDNA was cloned and the sequence was determined.

### Cloned Peptides and Derivatives thereof Binding to HSA in the Display Form

mRNAs of p1 and p2 were synthesized from the colony PCR product. The reverse or shuffled sequences of the mRNAs of the p1 and p2 peptides were prepared as follows. The DNA template was prepared using T7SD8M2.F44 as the forward primer, and SD8No38revG5S4.R69, SD8No38ranG5S4.R69, SD8No41revG5S4.R75 or SD8No41ranG5S4.R75 as the reverse primer. An elongation PCR and transcription were performed by a method similar to the method used in preparation of T7-mRNA. mRNA was purified by phenol/chloroform extraction and 2-propanole precipitation, and dissolved in ultrapure water.

Translation (2 µL), reverse transcription (4 µL), and the quenching reaction (5.25 µL) were performed by the above method.

2 µL of the obtained solution was mixed with HSA immobilized SA beads (0.3 pmol HSA) or SA-beads. After incubation at room temperature for 10 min., the beads were washed three times with 10 µL of HBST. In the second washing, 100 µL of HBST was used to perform stringent washing at 37°C for 30 min. The recovered cDNA was quantified with real-time PCR.

### SEQUENCE LISTING FREE TEXT

<210> is the SEQ ID NO. <223> is other information.
<210> 1
   < 223> T7SD8M2.F44
<210> 2
   <223> G5S-4an21.R41
<210> 3
   < 223> G5S-4an13.R36
<210> 4
   < 223> G5S-4.R20
<210> 5
   < 223> Biotin-DNA
<210> 6
   < 223> Competitor DNA
<210> 7
   < 223> Puromycin-linker.an13
< 210> 8
   < 223> Puromycin-linker.an21
<210> 9
   < 223> SD8-NNK
<210> 10
   < 223> NNK-CG5S4
<210> 11
   < 223> SD8T7G5S4.R72
<210> 12
   < 223> SD8No38revG5S4.R69
< 210> 13
   < 223> SD8No38ranG5S4.R69
<210> 14
   < 223> SD8No41revG5S4.R75
<210> 15
   < 223> SD8No41ranG5S4.R75
<210> 16
   < 223> C+GGGGGS spacer
<210> 17
   < 223> C + spacer peptide
<210> 18
   < 223> An21 mRNA annealing sequence
< 210> 19
   < 223> spacer and annealing sequence
< 210> 20
   < 223> stop and annealing sequence to An 13
< 210> 21
   < 223> p1
< 210> 22
   < 223> p2
< 210> 23
   < 223> p3
<210> 24
   < 223> p4
<210> 25
   < 223> p5
<210> 26
   < 223> p6
< 210> 27
   < 223> p7
<210> 28
   < 223> p8
<210> 29
   < 223> p9
<210> 30
   < 223> Reversed p1
< 210> 31
   < 223> Shuffled p1
<210> 32
   < 223> Reversed p2
<210> 33
   < 223> Shuffled p2
<210> 34
   < 223> 5'-SD-AUG (Ex.5)
<210> 35
   <223> AUG-spacer-an21 (Ex.5)
<210> 36
   < 223> spacer (Ex.5)

## Claims

1. A transcription-linker association-translation coupling reaction system **characterized by** incorporation of a template DNA library to enable a step of forming translation product/linker/mRNA complexes through transcription of a template DNA library to mRNAs, association of the mRNAs with linkers, translation of the mRNAs, and binding with the translation products to be automatically performed in a reaction system, comprising factors necessary for transcription, factors necessary for translation, and linkers.

2. The reaction system according to Claim 1, wherein the step of forming translation product/linker/mRNA complexes through transcription of a template DNA library to mRNAs, association of the mRNAs with linkers, translation of the mRNAs, and binding with the translation products is completed in an hour.

3. The reaction system according to either Claim 1 or 2, wherein the reaction system includes EF-P.

4. The reaction system according to any one of Claims 1 to 3, wherein the linkers each contain a section that forms a complex with an mRNA in a translation system; a base sequence of a linker in said section is annealed with a sequence of a 3'-end untranslated region of an mRNA; and an mRNA and a linker are bound through a covalent bond and/or a noncovalent bond.

5. The reaction system according to Claim 4, wherein a linker and each mRNA are bound by a noncovalent bond.

6. The reaction system according to either Claim 4 or 5, wherein a complementarity of first 10 bases on a 3'-end of a base sequence of a linker in the section that forms a complex with an mRNA and a sequence of a 3'-end untranslated region of an mRNA is 50% or higher.

7. The reaction system according to any one of Claims 1 to 6, wherein the linkers include a nucleophilic reagent that can be bound to a translation product.

8. The reaction system according to any one of Claims 1 to 7, wherein each DNA in the library contains a promoter sequence of transcriptase, and the reaction system includes transcriptase at 0.1 µM or higher.

9. A method for producing a library of translation products by incorporation of a template DNA library to the reaction system according to any one of Claims 1 to 8.

10. A method for producing a nucleic acid library that encodes translation products having desired functions, wherein
(a) incorporating a template nucleic acid library to the reaction system according to any one of Claims 1 to 8 to create a library of translation product/linker/mRNA complexes;
(b) selecting a translation product having a desired function from the library of translation product/linker/mRNA complexes;
(c) amplifying an mRNA or a cDNA binding with a selected translation product having a desired function to obtain a nucleic acid library.

11. The method according to claim 10, wherein the translation product having a desired function is a translation product binding to a target.

12. A method for obtaining DNA that encodes a translation product binding to a target, comprising
(a) incorporating a template DNA library to the reaction system according to any one of Claims 1 to 8 to create a library of translation product/linker/mRNA complexes;
(b) performing reverse transcription of mRNA sections in the translation product/linker/mRNA complexes to obtain a library of translation product/linker/mRNA complexes;
(c) selecting a translation product binding to a target from the library of translation product/linker/mRNA complexes;
(d) amplifying a cDNA binding to a selected translation product.

13. The method according to Claim 12, wherein step (d) includes recovering a cDNA from translation product/linker/mRNA-cDNA complexes, and amplifying a recovered cDNA by PCR; and
using obtained PCR products in their original state without purification as a template DNA library to further perform steps (a) to (e).

14. The method according to either Claim 12 or 13, wherein steps (a) to (e) can be performed within 3 hours.

15. The method according to either Claim 12 or 13, wherein steps (a) to (e) are repeated multiple times.

16. The method according to Claim 15, wherein steps (a) to (e) are repeated twice or more per day.

17. The method according to any one of Claims 10-16 that is automated by machine operation.
